# EUROPÄISCHE PATENTSCHRIFT

(11) **EP 2 399 985 B1**
(45) Veröffentlichungstag und Bekanntmachung des Hinweises auf die Patenterteilung: **29.04.2020**
(21) Anmeldenummer: 10015997.9
(22) Anmeldetag: 23.12.2010
(51) Int. Cl.: C12M 1/42, C12M 3/00

(54) **Verfahren und Vorrichtung zur gleichmässigen Behandlung von adhärenten Zellen**
Method and electrode assembly for treating adherent cells
Procédé et agencement d'électrodes pour le traitement de cellules adhérentes

(30) Priorität: 22.06.2010 EP 10006458
(43) Veröffentlichungstag der Anmeldung: 28.12.2011
(73) Patentinhaber: Lonza Cologne GmbH, 50829 Köln (DE)
(72) Erfinder: Koblizek, Thomas, 37081 Göttingen (DE); Heinze, Andreas, 50739 Köln (DE); Gleissner, Timo, 53879 Euskirchen (DE); Müller-Hartmann, Herbert, 50937 Köln (DE)
(74) Vertreter: Remus, Alvaro Johannes

(56) Entgegenhaltungen:
- WO-A1-00/23563
- WO-A1-2005/075656
- WO-A1-2009/140161
- WO-A1-2010/117806
- WO-A2-2007/094947
- US-A1- 2005 089 991
- US-A1- 2007 105 214
- US-A1- 2009 305 380
- US-B1- 6 352 853
- ANONYMOUS: "Petri-Pulser PP35-2P - Technical information", , [Online] 9. November 2010 (2010-11-09), XP002609839, BTX Harvard Apparatus Holliston (US) Gefunden im Internet: URL:http://www.btxonline.com/products/elec trodes/celltransfection/> [gefunden am 2010-11-09]
- DATABASE WPI Week 200449 Thomson Scientific, London, GB; AN 2004-514495 XP002610220, & JP 2004 202086 A (TEIKOKU SEIYAKU KK) 22. Juli 2004 (2004-07-22)
- LIN ET AL.: "Simulation and experimental demonstration of the electric field assisted electroporation microchip for in vitro gene delivery enhancement", LAB CHIP, vol. 4, 31 December 2004 (2004-12-31), - 31 December 2004 (2004-12-31), pages 104-108,

## Beschreibung

### Hintergrund der Erfindung

Die Erfindung betrifft ein Verfahren zur Beaufschlagung von adhärenten Zellen, die am Boden eines Reaktionsraums haften, mit mindestens einem elektrischen Feld, bei dem das elektrische Feld durch das Anlegen einer Spannung an mindestens zwei aktive Elektroden erzeugt wird, wobei mindestens drei Elektroden vorgesehen sind, von denen beim Anlegen der Spannung zur Erzeugung eines ersten elektrischen Feldes mindestens zwei Elektroden aktive Elektroden sind, und wobei mindestens ein zweites elektrisches Feld erzeugt wird. Die Erfindung betrifft ferner eine Vorrichtung, die mindestens drei Elektroden umfasst, welche über mindestens eine Schaltvorrichtung mit mindestens einer Spannungsquelle verbunden sind.

### Stand der Technik

Die Beaufschlagung von lebenden Zellen mit einem elektrischen Feld bzw. Spannungsimpuls, die so genannte Elektroporation bzw. Elektrotransfektion, wird seit Jahren auf Zellen in den verschiedensten Zuständen angewandt. Als Einzelzellen in Suspension in einer Pufferlösung, im adhärenten Zustand in einem Kulturgefäß, meist am Boden eines Kunststoffbehälters und in vivo, wo Zellen in der Regel im Gewebeverband in eine extrazelluläre Matrix eingebettet sind. Grundsätzlich werden bei der Elektroporation die Fremdmoleküle aus einer an die Zellen angepassten Pufferlösung oder einem Zellkulturmedium durch einen kurzzeitigen Stromfluss in die Zellen eingebracht, wobei durch die Wirkung der elektrischen Spannungsimpulse bzw. des dadurch entstehenden elektrischen Feldes und Stromflusses die Zellmembran für die Fremdmoleküle durchlässig gemacht wird. Die Zellsuspension befindet sich dabei häufig in einer sogenannten Küvette, d.h. einem schmalen, offenen Gefäß, dessen Probenraum zwei gegenüberliegende, annähernd parallele Elektroden in den Seitenwänden aufweist, welche zum Anlegen der elektrischen Spannung dienen. Durch die kurzzeitig entstehenden "Poren" in der Zellmembran gelangen die biologisch aktiven Moleküle zunächst in das Zytoplasma, in dem sie ggf. bereits ihre zu untersuchende Funktion ausüben können, und daraufhin unter bestimmten Bedingungen auch in den Zellkern. Durch das kurzzeitige Anlegen eines starken elektrischen Feldes, d.h. eines kurzen Spannungsimpulses mit hoher Stromdichte, können darüber hinaus auch Zellen, Zellderivate, subzelluläre Partikel und/oder Vesikel fusioniert werden. Bei dieser sogenannten Elektrofusion werden die Zellen beispielsweise zunächst durch ein inhomogenes elektrisches Wechselfeld in engen Membrankontakt gebracht. Durch das anschließende Anlegen eines elektrischen Feldimpulses kommt es dann zur Interaktion von Membranteilen, die schließlich zur Fusion führt. Für die Elektrofusion können dabei vergleichbare apparative Vorrichtungen verwendet werden, wie für die Elektroporation. Darüber hinaus können lebende Zellen durch elektrische Felder auch in einer ihre Eigenschaften verändernden Weise stimuliert werden.

Aus der WO 2005/056788 A1 ist beispielsweise ein Verfahren zur Elektroporation bekannt, bei dem Zellen auf einer mikroporösen Membran wachsen, die sich zwischen zwei parallel angeordneten Elektrodenflächen befindet.

Die US-A-5 134 070 beschreibt Anwendungen und Vorrichtungen zur Elektroporation von Zellen, die auf einer elektrisch leitenden Oberfläche wachsen, welche als Elektrode dient. Das Kulturgefäß wird von oben mit einer plattenförmigen Gegenelektrode abgedeckt, wobei ein Spalt gebildet wird, über den elektrische Entladungen möglich sind.

Aus der WO 2008/104086 A1 ist ferner eine Vorrichtung bekannt, bei der die Zellen auf co-planaren Elektrodenflächen wachsen. Der elektrische Kontakt zwischen den Elektroden wird über das Zellkulturmedium über den Zellen hergestellt, wobei die zwei Elektrodenbereiche durch eine isolierende Barriere getrennt sind, die aber trotzdem eine Elektrolytbrücke zwischen den Elektroden zulässt. Diese können beispielsweise aus Indiumzinnoxid bestehen, das als transparenter Halbleiter eine mikroskopische Analyse der Zellen ermöglicht.

Aus der WO 2009/131972 A1 ist eine Vorrichtung zur Elektroporation von Zellen bekannt, welche adhärent auf einer runden, scheibenförmigen Platte wachsen. Die Vorrichtung weist zwei parallel zueinander angeordnete Elektroden auf, wobei eine Elektrode sich auf der konkaven Oberfläche eines außen liegenden Zylinders und die andere Elektrode sich auf der konvexen Oberfläche eines innen liegenden Zylinders befindet.

Aus der US 2009/0305380 A1 ist ferner eine Vorrichtung zur Elektroporation von Zellen bekannt, die auf einer festen Fläche immobilisiert sind. Das elektrische Feld, mit dem die Zellen beaufschlagt werden, wird durch eine Anordnung von Elektrodenpaaren erzeugt, die sich dicht nebeneinander liegend auf einer oberhalb der festen Fläche angeordneten Oberfläche befinden. Die Elektroden werden durch elektrische Bahnen gebildet, die auf die Oberfläche aufplattiert sind. Die beiden Elektroden eines Elektrodenpaars sind dabei so dicht nebeneinander angeordnet, dass sich nicht mehr als eine Zelle innerhalb des geringsten Abstandes zwischen den beiden Elektroden befinden kann.

Lin et al. (LAB CHIP, Bd. 4, 31.12.2004, 104-108) beschreiben ein Verfahren und einen Mikrochip zur Elektroporation von adhärenten Zellen. Der Mikrochip weist eine Vielzahl von ineinandergreifenden Mikroelektroden auf und ist an eine Spannungsquelle anschließbar. Die Mikroelektroden, die von den Zellen überwachsen werden, können in zwei Gruppen (Anoden und Kathoden) eingeteilt werden, wobei zusätzlich noch eine weitere Elektrode (Kathode) oberhalb des Mikrochips angeordnet ist. Zunächst wird ein elektrisches Feld zwischen der weiteren Elektrode und einigen Mikroelektroden erzeugt, um eine Anreicherung von Plasmiden im Bereich der Zellen zu erreichen. Dann wird die eigentliche Elektroporation im Bereich der Zellen durch Erzeugen eines elektrischen Felds zwischen den Mikroelektroden durchgeführt.

Die WO 2010/117806 A1 offenbart ein Verfahren zur Behandlung von Läsionen innerhalb eines menschlichen Körpers, bei dem Spannungsimpulse im Bereich einer Läsion erzeugt werden, um diese mittels irreversibler Elektroporation zu zerstören. Elektroden in Form von Sonden werden zu diesem Zweck unter visueller Kontrolle in den Körper des Patienten und insbesondere in unmittelbare Nähe der Läsion eingeführt. Dabei werden die einzelnen Elektroden, die separat an einen Generator angeschlossen sind, jeweils individuell gesteuert.

Aus der WO2009/140161 A1 ist ferner eine Vorrichtung zur Elektroporation von adhärenten Zellen bekannt, die eine Anordnung von dicht nebeneinander angeordneten Elektroden umfasst. Die Elektroden sind dabei jeweils alternierend an den Plus- und den Minuspol einer Spannungsquelle angeschlossen.

Die Firma BTX vertreibt mit dem PetriPulser® eine Anordnung von abwechselnd gepolten planparallelen Elektrodenplatten, die senkrecht auf adhärent in einem Kulturgefäß wachsende Zellen aufgebracht werden kann. Dabei tauchen die Elektroden in den Kulturüberstand ein, wobei sich die Zwischenräume zwischen den einzelnen Elektrodenplatten mit dem Kulturmedium füllen. Ein wesentlicher Nachteil dieser Anordnung liegt darin, dass der Großteil des Stromes über das über den Zellen befindliche zellfreie Kulturmedium abfließt. Das Feld ist aber nur wirksam im Randfeld am Boden des Gefäßes, wo sich die Zellen befinden, so dass unnötig hohe Ströme zu erbringen sind. Ferner muss von einer hohen Mortalität der Zellen durch pH-Wert-Änderungen und hohen Strom ausgegangen werden. Darüber hinaus muss die Spannungsversorgung für lang anhaltende Spannungsimpulse sehr groß ausgelegt werden, um diese großen Ströme und damit Ladungsmengen und Leistungen zu erbringen. Zudem muss ein großes Volumen aufgebracht werden, das für die Elektroporation geeignet ist und das zu transfizierende Substrat in ausreichend hoher Konzentration enthält, wodurch auch die Menge an Substrat entsprechend höher liegt. Ein weiterer wesentlicher Nachteil dieser bekannten Vorrichtung liegt darin, dass das elektrische Feld im Bereich orthogonal unterhalb der freien Elektrodenspitzen minimal ist. Dies führt dazu, dass die Zellen, die sich in diesem Bereich befinden, praktisch nicht transfiziert oder fusioniert werden, so dass die Effizienz der mit dieser bekannten Vorrichtung durchgeführten Verfahren insgesamt gering ist.

### Beschreibung der Erfindung

Der Erfindung liegt die Aufgabe zugrunde, ein Verfahren und eine Vorrichtung zu schaffen, die eine effiziente und gleichmäßige Behandlung von adhärenten Zellen mit einem elektrischen Feld ermöglichen.

Die Aufgabe wird erfindungsgemäß durch ein Verfahren der eingangs genannten Art gelöst, bei welchem beim Anlegen der Spannung zur Erzeugung des zweiten elektrischen Feldes mindestens eine der zwei zuvor aktiven Elektroden eine potentialfreie Elektrode ist, wobei so viele elektrische Felder erzeugt werden, dass alle beim Erzeugen des ersten elektrischen Feldes aktiven Elektroden während des Erzeugens der weiteren elektrischen Felder jeweils mindestens einmal potentialfreie Elektroden sind. Wenn beispielsweise eine erste Elektrode beim Beaufschlagen der Zellen mit einem Spannungsimpuls aktiv ist, also entweder Plus- oder Minuspol, dann ist das elektrische Feld unterhalb dieser Elektrode minimal, so dass die orthogonal unterhalb dieser Elektrode befindlichen Zellen nicht ausreichend elektrisch behandelt werden. Erfindungsgemäß wird daher ein zweites elektrisches Feld erzeugt, wobei die genannte erste Elektrode nun potentialfrei (passiv) ist, d. h. das elektrische Feld wird durch das Anlegen einer Spannung an mindestens zwei andere Elektroden erzeugt. Hierdurch wird erreicht, dass nunmehr während des zweiten Spannungsimpulses auch der Bereich unterhalb der besagten ersten Elektrode mit dem elektrischen Feld beaufschlagt wird. Folglich wird durch das erfindungsgemäße Verfahren gewährleistet, dass auch die Zellen, die während des ersten Spannungsimpulses keinem elektrischen Feld ausgesetzt waren, ebenfalls ausreichend elektrisch behandelt werden. Mit Hilfe des erfindungsgemäßen Verfahrens kann also die Anzahl der Bereiche, innerhalb derer das elektrische Feld gering ist, effektiv minimiert werden, so dass die Effizienz das jeweiligen Verfahrens, beispielsweise einer Elektrotransfektion oder Elektrofusion, optimiert werden kann. Das erfindungsgemäße Verfahren ist folglich in vorteilhafter Weise dazu geeignet, eine effiziente und gleichmäßige Behandlung von adhärenten Zellen mit einem elektrischen Feld zu gewährleisten. Dadurch, dass alle beim Erzeugen des ersten elektrischen Feldes aktiven Elektroden während des Erzeugens der weiteren elektrischen Felder jeweils mindestens einmal potentialfreie Elektroden sind, ist sichergestellt, dass unterhalb der Elektroden keine Bereiche mehr existieren, die nicht mit einem elektrischen Feld beaufschlagt wurden. Folglich werden in diesem optimalen Fall alle Zellen gleichmäßig mit einem elektrischen Feld behandelt, so dass für das jeweilige Verfahren, beispielsweise eine Elektrotransfektion oder Elektrofusion, eine maximale Effizient gewährleistet werden kann.

In vorteilhafter Ausgestaltung der Erfindung ist vorgesehen, dass jedes elektrische Feld jeweils durch mindestens einen Spannungsimpuls, vorzugsweise mindestens zwei Spannungsimpulse, erzeugt wird. Die elektrischen Felder können erfindungsgemäß jeweils durch einen einzelnen Spannungsimpuls oder mindestens zwei bzw. mehrere kurz aufeinander folgende oder ineinander übergehende (Teil-) Spannungsimpulse erzeugt werden. Die Teilimpulse können dabei durch die Entladung eines oder mehrerer Ladungsspeicher, vorzugsweise eines Kondensators oder mindestens zweier Kondensatoren, erzeugt werden. Vorzugsweise ist jeder Spannungsimpuls, der ein elektrisches Feld erzeugt, ein Doppelimpuls, der sich aus einem kurzen Hochspannungsimpuls und einem längeren Impuls mit relativ geringer Spannung zusammensetzt. Solch ein Doppelimpuls kann beispielsweise durch die kurz aufeinander folgende Entladung zweier Kondensatoren erzeugt werden, wobei die beiden Teilimpulse ohne Unterbrechung ineinander übergehen können. Beispielsweise kann eine erste Ladungsspeichereinrichtung eine Kapazität von mindestens 10 µF, vorzugsweise mindestens 50 µF, insbesondere mindestens 100 µF, aufweisen und/oder mit einer Anfangsspannung von 10 bis 1500 V, vorzugsweise 20 bis 1200 V, insbesondere 50 bis 1000 V, bei einer Pulsdauer von 2 µs bis 40 ms, vorzugsweise 5 µs bis 20 ms, insbesondere 10 µs bis 10 ms, entladen werden. Kurz vor, gleichzeitig mit oder kurz nach dem Ende der Entladung der ersten Ladungsspeichereinrichtung kann eine zweite Ladungsspeichereinrichtung, die beispielsweise eine Kapazität von mindestens 100 µF, vorzugsweise mindestens 500 µF, insbesondere mindestens 1000 µF, aufweisen kann, mit einer Anfangsspannung von beispielsweise 10 bis 240 V, vorzugsweise 20 bis 200 V, insbesondere 50 bis 150 V, bei einer Pulsdauer von beispielsweise 1 ms bis 500 ms, vorzugsweise 2 ms bis 350 ms, insbesondere 4 ms bis 250 ms, entladen werden.

Gleichzeitig oder alternativ können die mindestens zwei elektrischen Felder jeweils durch mindestens einen Spannungsimpuls erzeugt werden, wobei der zeitliche Abstand zwischen den Spannungsimpulsen mindestens 500 ms, vorzugsweise mindestens 1 s, insbesondere 1 bis 10 s, beträgt. Der zeitliche Abstand zwischen den Spannungsimpulsen (nicht Teilimpulsen!), die jeweils ein elektrisches Feld im Sinne der Erfindung erzeugen, muss also überraschender Weise relativ groß sein, damit die Überlebensrate der Zellen stabil bleibt und/oder eine hohe Effizienz des Verfahrens gewährleistet werden kann. Jeder einzelne Spannungsimpuls kann sich dabei wie oben beschrieben auch aus mehreren, d. h. mindestens zwei, Teilimpulsen zusammensetzen.

In weiterer vorteilhafter Ausgestaltung der Erfindung ist vorgesehen, dass mehrere Reaktionsräume vorgesehen sind, in denen jeweils adhärente Zellen mit den elektrischen Feldern beaufschlagt werden, wobei der zeitliche Abstand zwischen zwei Spannungsimpulsen zur Erzeugung zweier elektrischer Felder in einem Reaktionsraum dazu genutzt wird, mindestens ein elektrisches Feld in mindestens einem weiteren Reaktionsraum zu erzeugen. Diese Ausgestaltung der Erfindung ist besonders vorteilhaft, wenn viele Proben innerhalb kürzester Zeit behandelt werden müssen, also insbesondere in Hochdurchsatzverfahren. Wie oben bereits beschrieben können die einzelnen Spannungsimpulse zur Erzeugung der elektrischen Felder in den jeweiligen Reaktionsräumen einen relativ großen zeitlichen Abstand voneinander haben. Bei der Behandlung vieler Proben würde dies jeweils zu einer sehr langen Prozessdauer führen. Erfindungsgemäß werden daher die einzelnen Spannungsimpulse in Bezug auf die unterschiedlichen zu behandelnden Reaktionsräume vorzugsweise verschachtelt erzeugt, so dass die Wartezeit zwischen der Erzeugung zweier elektrischer Felder in einem Reaktionsraum zur Erzeugung mindestens eines elektrischen Feldes in mindestens einem weiteren Reaktionsraum genutzt werden kann. Auf diese Weise lässt sich die gesamte Prozessdauer, beispielsweise bei der Behandlung aller Reaktionsräume einer "24-well" Zellkulturplatte, deutlich reduzieren. Darüber hinaus wird durch dieses Vorgehen die Gefahr verringert, dass sich zwischen den Proben in den unterschiedlichen Reaktionsräumen Abweichungen aufgrund unterschiedlicher Inkubationszeiten vor und/oder nach der Behandlung ergeben. Würden beispielsweise die Zellen in den einzelnen Reaktionsräumen einer "24-well" Platte über einen längeren Zeitraum nacheinander behandelt, so würden die Proben in den ersten Reaktionsräumen eine relativ lange Inkubationszeit nach der Behandlung durchlaufen, während die zuletzt behandelten Proben eine längere Inkubationszeit vor der Behandlung durchlaufen müssten. Dies kann die Effizienz der Behandlung in den einzelnen Reaktionsräumen beeinflussen und letztendlich zu unterschiedlichen Ergebnissen bei den einzelnen Proben führen. Das verschachtelte Behandeln der unterschiedlichen Reaktionsräume führt dagegen aufgrund der Minimierung unterschiedlicher Inkubationszeiten zu gleichmäßigeren und damit besser reproduzierbaren Ergebnissen bzw. Effizienzen.

In besonders vorteilhafter Ausgestaltung der Erfindung ist ferner vorgesehen, dass die Elektroden und die adhärenten Zellen voneinander beabstandet sind und dass der Abstand zwischen den Zellen und den Elektroden auf einen empirisch ermittelten Wert eingestellt wird. Vorzugsweise wird dabei der Abstand so eingestellt, dass die Stärke des elektrischen Feldes zwischen den aktiven Elektroden und unterhalb der potentialfreien Elektroden jeweils optimiert ist. Es konnte gezeigt werden, dass die Dichte der Feldlinien mit größer werdendem Abstand abnimmt, so dass das effektive elektrische Feld auf der Bodenfläche des Gefäßes mit größer werdendem Abstand geringer wird. Dieser Effekt könnte im Prinzip durch eine Erhöhung der angelegten Spannung, d. h. eine Erhöhung der Stromdichte, kompensiert werden. Dies würde allerdings den technischen Aufwand und das Risiko von Blitzentladungen erhöhen. Zu geringe Abstände sind ebenfalls nicht sinnvoll, da dann bereits geringe Unebenheiten der Bodenfläche zu einer Inhomogenität des elektrischen Feldes und somit zu nicht reproduzierbaren Ergebnissen führen würden. Es ist in diesem Zusammenhang ferner zu berücksichtigen, dass die Feldstärke zwischen den aktiven Elektroden mit größer werdendem Abstand abnimmt, während die Feldstärke unterhalb der potentialfreien Elektroden bei einem mittleren Abstand einen Maximalwert erreicht, also ausgehend von diesem Maximalwert bei kleiner oder größer werdenden Abständen abnimmt. Der Abstand der Elektroden zu den Zellen muss daher in Abhängigkeit von den Reaktionsbedingungen derart eingestellt werden, dass die Feldstärke unterhalb der potentialfreien Elektroden dem Maximalwert möglichst nahe kommt und gleichzeitig zwischen den aktiven Elektroden einen möglichst hohen Wert erreicht. Dieser optimale Abstand muss für die unterschiedlichen Bedingungen, wie beispielsweise Geometrie des Reaktionsraums, Art der verwendeten Pufferlösung, Form und Größe der Elektroden und elektrische Parameter, empirisch ermittelt werden.

Die Aufgabe wird erfindungsgemäß ferner durch eine Vorrichtung der eingangs genannten Art gelöst, insbesondere einer Vorrichtung zur Durchführung des erfindungsgemäßen Verfahrens, bei der mindestens fünf Elektroden über vier Schaltvorrichtungen mit der mindestens einen Spannungsquelle verbunden sind, wobei mindestens zwei Elektroden über eine gemeinsame Schaltvorrichtung mit der Spannungsquelle verbunden sind, und wobei zwei Gruppen von Elektroden mit jeweils mindestens zwei Elektroden vorgesehen sind, die jeweils über zwei gemeinsame Schaltvorrichtungen mit der Spannungsquelle verbunden sind. Aufgrund dieser besonders vorteilhaften Konfiguration können alle Elektroden entweder aktiv oder passiv (potentialfrei) geschaltet werden. Bei der erfindungsgemäßen Vorrichtung wird durch das Gruppieren von Elektroden und die Begrenzung der Anzahl der Schaltvorrichtungen der apparative und konstruktive Aufwand minimiert, so dass eine kostengünstige und wenig störanfällige Vorrichtung zur Verfügung gestellt wird. In Verbindung mit dem oben beschriebenen Verfahren ermöglicht die erfindungsgemäße Vorrichtung eine effiziente und gleichmäßige Behandlung von adhärenten Zellen mit einem elektrischen Feld, wobei das Umschalten zwischen aktiven und potentialfreien Elektroden mit einer geringen Anzahl von Schaltvorrichtungen schnell und sicher durchgeführt werden kann. Dadurch, dass zwei Gruppen von Elektroden vorgesehen sind, die jeweils über zwei gemeinsame Schaltvorrichtungen mit der Spannungsquelle verbunden sind, kann eine relativ große Anzahl von Elektroden durch lediglich vier Schaltvorrichtungen gesteuert werden, d. h. beide Elektrodengruppen können jeweils aktiv oder passiv (potentialfrei) geschaltet werden.

Vorzugsweise umfasst jede Gruppe mindestens drei Elektroden, besonders bevorzugt mindestens vier Elektroden. Insgesamt können in vorteilhafter Ausgestaltung der Erfindung mindestens sieben, vorzugsweise 9-21, Elektroden vorgesehen sein. Beispielsweise könnte erfindungsgemäß eine Konfiguration eingesetzt werden, bei der eine erste Gruppe 8 Elektroden und die zweite Gruppe 9 Elektroden umfasst.

In vorteilhafter Ausgestaltung der erfindungsgemäßen Vorrichtung ist vorgesehen, dass die Elektroden zum Einsetzen in mindestens einen Reaktionsraum vorgesehen sind, d. h. die Elektroden können beispielsweise derart angeordnet und als Einheit konstruiert sein, dass sie von oben in ein Zellkulturgefäß eingebracht werden und ggf. in das sich darin befindende Zellkulturmedium eintauchen können. Die Elektroden lassen sich dann in vorteilhafter Weise oberhalb der Zellen anordnen, die auf dem Boden des Reaktionsraums haften. Vorzugsweise ist der Reaktionsraum dabei Teil einer Multiwell-Platte, so dass eine Vielzahl von Proben unter vergleichbaren Bedingungen mit der erfindungsgemäßen Vorrichtung behandelt werden können.

Bei den Schaltvorrichtungen kann es sich beispielsweise um Leistungshalbleiter, vorzugsweise IGBTs oder MOSFETs, oder elektromechanische Vorrichtungen, vorzugsweise Relais, handeln.

Die Erfindung umfasst auch die Verwendung der erfindungsgemäßen Vorrichtung zum Beaufschlagen von adhärenten Zellen mit mindestens einem elektrischen Feld, insbesondere zur Elektroporation oder Elektrofusion von adhärenten Zellen.

Die Erfindung wird im Folgenden anhand der Abbildungen beispielhaft näher erläutert.

### Kurze Beschreibung der Abbildungen

Figur 1 zeigt eine schematische Seitenansicht einer Elektrodenanordnung einer beispielhaften erfindungsgemäßen Vorrichtung mit drei Elektroden.
Figur 2 zeigt eine schematische Seitenansicht einer alternativen Elektrodenanordnung einer beispielhaften erfindungsgemäßen Vorrichtung mit vier Elektroden.
Figur 3 zeigt eine perspektivische Ansicht der Unterseite einer beispielhaften Ausführungsform einer beispielhaften Elektrodenanordnung für eine erfindungsgemäße Vorrichtung.
Figur 4 zeigt eine weitere perspektivische Ansicht der Elektrodenanordnung gemäß Figur 3, wobei in dieser Darstellung die innen liegenden Teile der Elektroden und die Kontaktelemente sichtbar sind.
Figur 5 zeigt eine perspektivische Ansicht der Oberseite der Elektrodenanordnung gemäß Figur 3.
Figur 6 zeigt einen Längsschnitt durch die Elektrodenanordnung gemäß den Figuren 3 bis 5.
Figur 7 zeigt in einem Balkendiagramm die Abhängigkeit der Transfektionseffizienz vom Abstand der Elektroden einer erfindungsgemäßen Elektrodenanordnung zu den auf der Kulturfläche anhaftenden Zellen bei drei unterschiedlich starken Spannungsimpulsen (x-Achse: Abstand [mm], y-Achse: Transfektionseffizienz [%], A-5 = schwacher Spannungsimpuls, K-19 = mittelstarker Spannungsimpuls, AX-19 = starker Spannungsimpuls).
Figur 8 zeigt ein Diagramm der relativen elektrischen Feldstärke in Abhängigkeit vom Abstand der Elektroden zum Boden des Gefäßes, bezogen auf die Feldstärke bei einem Abstand von 500 µm (x-Achse: Abstand [µm], y-Achse: relative Transfektionseffizienz [%]). Dargestellt sind hier die relative Feldstärke zwischen aktiven Elektroden (graue Rauten) und die relative Feldstärke unterhalb der potentialfreien (passiven) Elektroden (schwarze Quadrate).
Figur 9 zeigt Simulationen der elektrischen Felder in einem Zellkulturgefäß bei ausschließlich aktiven Elektroden (A) sowie bei aktiven und potentialfreien Elektroden (B).

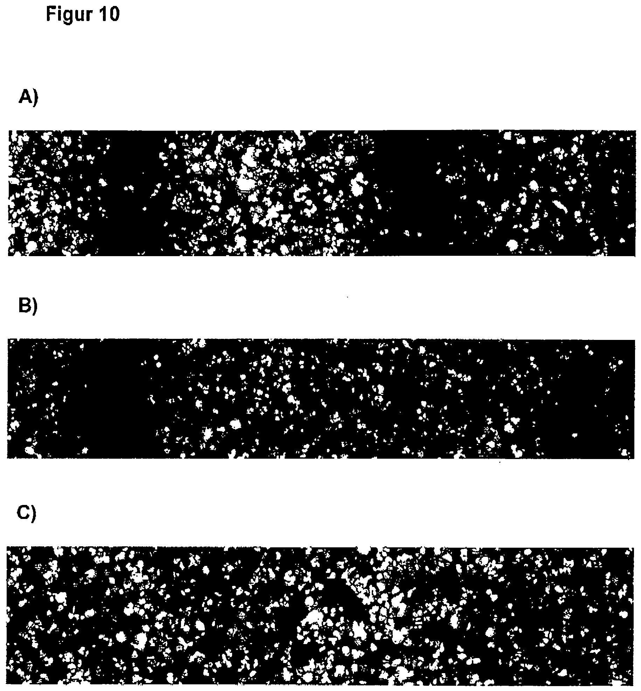

Figur 10 zeigt fluoreszenz-mikroskopische Bilder von Zellen, die mit einem Plasmid transfiziert wurden (Elektroporation mit Nucleofector®, Lonza), das für das fluoreszierende Protein MAXGFP® (Lonza) kodiert. Oben: A) Alle Elektroden waren während der Elektrotransfektion aktiv. Mitte: B) Gleiche Bedingungen wie oben, aber nur die Hälfte der Elektroden war während der Transfektion aktiv, die andere Hälfte potentialfrei. Unten: C) Dieselben Zellen wurden nochmals mit einem zweiten elektrischen Feld (Spannungsimpuls) beaufschlagt, jedoch waren beim zweiten Impuls aktive und passive Elektroden vertauscht, d. h. alle beim erstem Impuls aktiven Elektroden waren beim zweiten Impuls potentialfrei, während die zuvor passiven Elektroden beim zweiten Impuls aktiv waren.
Figur 11 zeigt fluoreszenz-mikroskopische Bilder von Zellen, die mit einem Plasmid, das für das fluoreszierende Protein MAXGFP® (Lonza) kodiert, gemäß dem erfindungsgemäßen Verfahren transfiziert wurden (Elektroporation mit Nucleofector®, Lonza). Oben: A) Abstand zwischen zwei Spannungsimpulsen < 0,5 s. Unten: B) Abstand zwischen zwei Spannungsimpulsen 2,0 s.
Figur 12 zeigt schematische Darstellungen der elektrischen Verschaltung einer Elektrodenanordnung mit 17 Elektroden, wobei in der rechts abgebildeten Darstellung (B) die in der links abgebildeten Darstellung (A) aktiven Elektroden potentialfrei (passiv) sind. Farblose Elektroden: potentialfrei (passiv). Gestrichelte Elektroden: aktiv (Hochspannung). Schwarze Elektroden: aktiv (Masse).
Figur 13 zeigt eine schematische Darstellung der elektrischen Verschaltung der Elektroden einer beispielhaften erfindungsgemäßen Vorrichtung mit vier Schaltvorrichtungen. Farblose Elektroden: potentialfrei (passiv). Gestrichelte Elektroden: aktiv (Hochspannung). Schwarze Elektroden: aktiv (Masse).
Figur 14 zeigt In einer Draufsicht schematische Darstellungen von Elektrodenanordnungen mit Kontaktpunkten. Die Elektrodenanordnungen weisen unterschiedlichen Abmessungen für Zellkulturplatten mit 3 größeren Reaktionsräumen ("6-well"-Format) und 12 kleineren Reaktionsräumen ("24-well"-Format) auf.

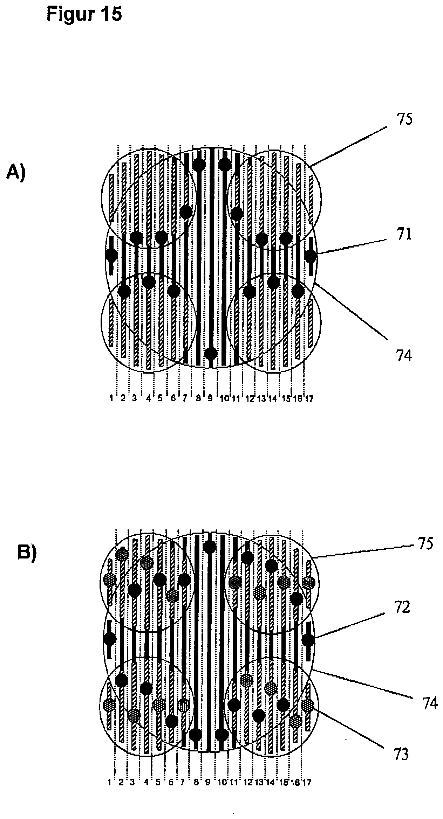

Figur 15 zeigt in einer Draufsicht schematische Darstellungen von Elektrodenanordnungen mit Kontaktpunkten. Die Zahlen 1 bis 17 unterhalb der Elektrodenanordnungen bezeichnen jeweils die Linien des Elektrodenrasters, innerhalb derer sich jeweils die Elektroden befinden. A) Eine beispielhafte und bevorzugte Anordnung der Kontaktpunkte für Elektrodenanordnungen mit unterschiedlichen Abmessungen für Zellkulturplatten mit mindestens einem größeren Reaktionsraum und mindestens vier kleineren Reaktionsräumen. B) Eine weitere beispielhafte und bevorzugte Anordnung der Kontaktpunkte für Elektrodenanordnungen mit unterschiedlichen Abmessungen für Zellkulturplatten mit mindestens einem größeren Reaktionsraum und mindestens vier kleineren Reaktionsräumen, bei der die Anordnung der Kontaktpunkte bzw. Kontaktelemente im Hinblick auf den Herstellungsprozess optimiert ist.
Figur 16 zeigt in einer Draufsicht eine schematische Darstellung einer weiteren beispielhaften und bevorzugten Anordnung der Kontaktpunkte für Elektrodenanordnungen mit unterschiedlichen Abmessungen für Zellkulturplatten mit 3 größeren Reaktionsräumen ("6-well"-Format) und 12 kleineren Reaktionsräumen ("24-well"-Format), bei der die Kontaktelemente um eine Achse drehbar sind.

### Beschreibung beispielhafter und bevorzugter Ausführungsformen der Erfindung

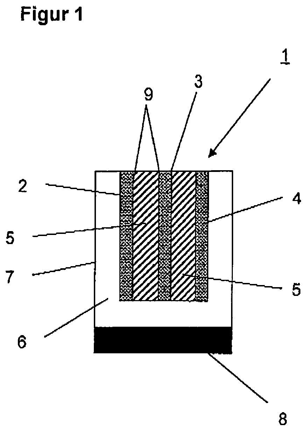

**Figur 1** zeigt eine erfindungsgemäße Elektrodenanordnung 1, die drei planparallel angeordneten Elektroden 2, 3, 4 umfasst, welche in den Innenraum 6 eines Gefäßes 7 hineinragen. Das Gefäß 7 umfasst eine Bodenfläche 8, auf der lebende Zellen anhaften und wachsen können (adhärente Zellen). Der Innenraum 6 ist üblicherweise mit einer Flüssigkeit gefüllt, beispielsweise einem Zellkulturmedium oder einer anderen an die Zellen adaptierten Lösung. Der Raum zwischen den Elektroden 2, 3, 4 ist vollständig mit einem elektrisch isolierenden Material 5 ausgefüllt, so dass beim Anlegen einer Spannung an die Elektroden 2, 3, 4 kein Strom über den zwischen den Elektroden 2, 3, 4 liegenden Raum abfließen kann. Der gesamte Strom fließt bei der erfindungsgemäßen Elektrodenanordnung 1 durch den Raum zwischen den Elektroden 2, 3, 4 und der Bodenfläche 8, so dass hier bei der Verwendung einer nicht-permanenten Spannungsquelle (z. B. Kondensator) die Spannung langsamer abfällt und somit die Feldstärke für die Behandlung der Zellen über die Zeit höher ist. Hierdurch kann einerseits die Vorrichtung zur Impulserzeugung sparsam dimensioniert werden und andererseits können in der Flüssigkeit stärkere Veränderungen des pH-Wertes vermieden werden, die ansonsten durch große geflossene Ladungsmengen infolge von Elektrolyse erzeugt würden.

In dem hier dargstellten Ausführungsbeispiel sind also planparallele Elektroden 2, 3, 4 durch isolierendes Material 5 getrennt, so dass die leitenden Oberflächen der Elektroden 2, 3, 4 nur nach unten (in Richtung der Bodenfläche 8 bzw. der darauf haftenden Zellen) frei sind und mit der Umgebung in elektrischem Kontakt stehen. Durch die volle Ausdehnung des isolierenden Materials 5 im Bereich zwischen den jeweils gegenüberliegend angeordneten Flächen 9 der planparallelen Elektroden 2, 3, 4, oder zumindest in dem für die Flüssigkeit offenen Bereich zwischen Elektroden, in dem diese parallele Linien beschreiben, ist das elektrische Feld fokussierbar bzw. der Strom auf den angestrebten Wirkbereich begrenzbar. Es ist ferner von besonderem Vorteil, dass eine Fokussierung des elektrischen Feldes im Bereich der adressierten Zellen bzw. eine Begrenzung des elektrischen Stromes auf den Wirkbereich nun auch bei Verwendung von planparallelen Elektroden 2, 3, 4 möglich ist, was über die Zeit konstante und stabilere Feldstärken und Stromdichten in dem adressierten Bereich zwischen den Elektroden 2, 3, 4 und der Bodenfläche 8 ermöglicht. Geeignete isolierende Materialien hierfür sind beispielsweise Platten oder Formspritzkörper aus gängigen, vorzugsweise thermoplastischen, Kunststoffen, wie beispielsweise Polyvinylchlorid, Polystyrol, Polypropylen, Polyethylen oder Polykarbonat. Durch den erfindungsgemäßen Aufbau kann der Abfluss von Strom über die sich jeweils gegenüberliegenden Flächen 9 der planparallelen Anteile der Elektroden 2, 3, 4 verhindert und somit Spannungsimpulse mit weniger abfallendem Stromniveau erzeugt werden. Eine erfindungsgemäße Anordnung kann also beispielsweise pro Reaktion, je nach Fläche des Kulturbodens mit den zu behandelnden Zellen, mit einer oder mehreren aufeinander folgenden Impulsentladungen geringerer Energie/Ströme beschickt werden, um die nötigen Leistungen pro Entladung zu begrenzen.

Es kann beispielsweise ein Elektroden-Isolator-Sandwich verwendet werden, bei dem die Elektroden abwechselnd gepolt sind. Bei einer solchen Anordnung ist das Feld in den Bereichen orthogonal unterhalb der aktiven Elektroden praktisch nicht vorhanden und daher nicht auf die dort vorzufindenden Zellen wirksam, die unterhalb der aktiven Elektroden liegen. Diese Bereiche befinden sich In unmittelbarer Nähe eines elektrischen Leiters (der Elektroden) und daher außerhalb eines nennenswerten Feldes. Aktive Bereiche, d. h. Bereiche mit einem ausreichenden elektrischen Feld, sind nur die Bereiche zwischen entgegengesetzt gepolten Elektroden. Um die Bereiche unterhalb der Elektroden, die nicht mit einem elektrischen Feld beaufschlagt werden, zu minimieren, können beispielsweise die Elektroden möglichst dünn ausgebildet sein (beispielsweise 50µm), so dass größere Bereiche der zellbewachsenen Bodenfläche von der Elektrodenanordnung mit aktiven Bereichen aus Elektroden-Isolator-Kombinationen bedeckt sind. Eine Verringerung der Elektrodendicke ist aber in vielen Fällen weder sinnvoll noch technisch machbar, so dass die Wirkung einer solchen Maßnahme limitiert ist.

Erfindungsgemäß wird daher nach dem ersten Spannungsimpuls mittels mindestens eines weiteren Spannungsimpulses mindestens ein weiteres elektrisches Feld im Raum zwischen den Elektroden 2, 3, 4 und der Bodenfläche 8 erzeugt. Dabei Ist erfindungsgemäß mindestens eine der zuvor aktiven Elektroden potentialfrei, so dass das weitere bzw. zweite elektrische Feld auch in dem Bereich unterhalb der jetzt passiven Elektrode auf die Zellen wirken kann. Wäre also in dem in Figur 1 dargestellten Ausführungsbeispiel beispielsweise die mittlere Elektrode 3 beim ersten Spannungsimpuls aktiv gewesen, also entweder mit Spannung (+) oder Masse (-) verbunden, so könnte diese Elektrode 3 beim zweiten Spannungsimpuls potentialfrei (passiv) bleiben, so dass dann auch der Bereich unterhalb der Elektrode 3, der gleichzeitig zwischen den während des zweiten Spannungsimpulses aktiven Elektroden 2 und 4 liegt, mit einem ausreichenden elektrischen Feld beaufschlagt werden. Auf diese Welse kann die Bodenfläche 8 gleichmäßiger abgedeckt und somit eine größere Anzahl von Zellen effektiv elektrisch behandelt werden.

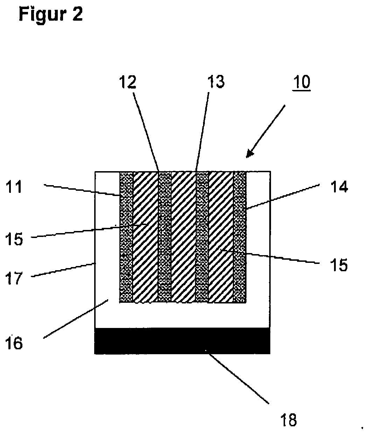

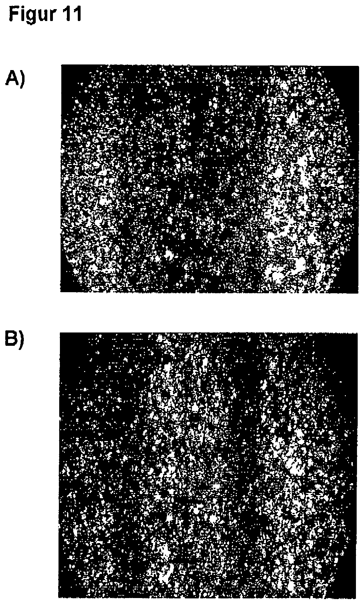

Figur 2 zeigt eine alternative Elektrodenanordnung 10 einer beispielhaften erfindungsgemäßen Vorrichtung mit vier Elektroden 11, 12, 13, 14. Die Elektrodenanordnung 10 entspricht im Wesentlichen der Elektrodenanordnung 1 gemäß Figur 1, mit dem Unterschied, dass diese vier planparallel angeordneten Elektroden 11, 12, 13, 14 umfasst, welche in den Innenraum 16 eines Gefäßes 17 hineinragen. Das Gefäß 17 umfasst ebenfalls eine Bodenfläche 18, auf der lebende Zellen anhaften und wachsen können (adhärente Zellen). Auch der Raum zwischen den Elektroden 11, 12, 13, 14 ist jeweils vollständig mit einem elektrisch isolierenden Material 15 ausgefüllt, so dass beim Anlegen einer Spannung an die Elektroden 11, 12, 13, 14 kein Strom über den zwischen den Elektroden 11, 12, 13, 14 liegenden Raum abfließen kann. Erfindungsgemäß können die Elektroden 11, 12, 13, 14 beispielsweise derart angesteuert werden, dass die Elektroden 11 und 13 bei einem ersten Spannungsimpuls aktiv sind, während die Elektroden 12 und 14 potentialfrei sind. Bei dem darauf folgenden Spannungsimpuls sind dann die zuvor aktiven Elektroden 11 und 13 potentialfrei, während die Elektroden 12 und 14 aktiv sind. Auf diese vorteilhafte Weise wird der gesamte Bereich zwischen den außen liegenden Elektroden 11 und 14, insbesondere jeweils auch der Bereich unterhalb der innen liegenden Elektroden 12 und 13, mit einem ausreichenden elektrischen Feld beaufschlagt. Somit wird die gesamte Bodenfläche 18 bzw. die darauf anhaftenden Zellen gleichmäßig elektrisch behandelt (siehe auch Figuren 10 und 11).

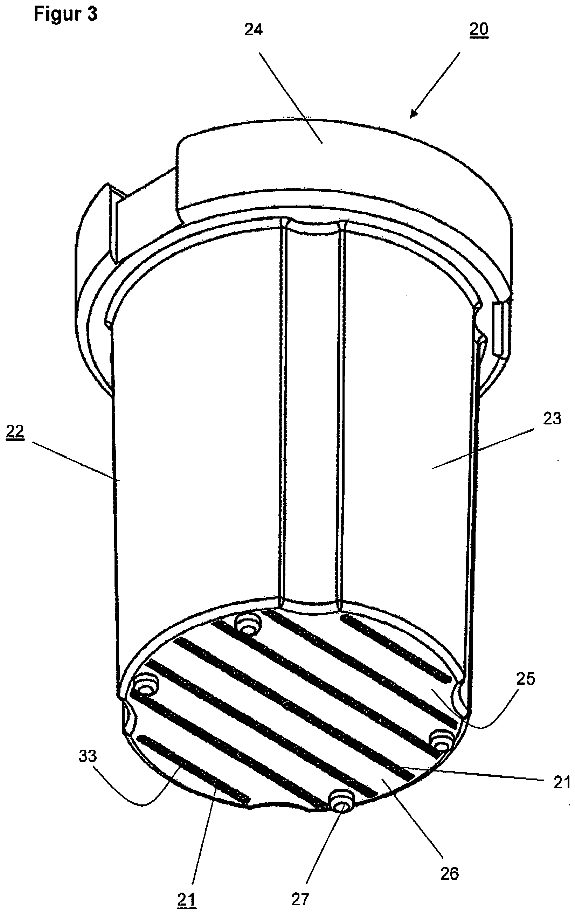

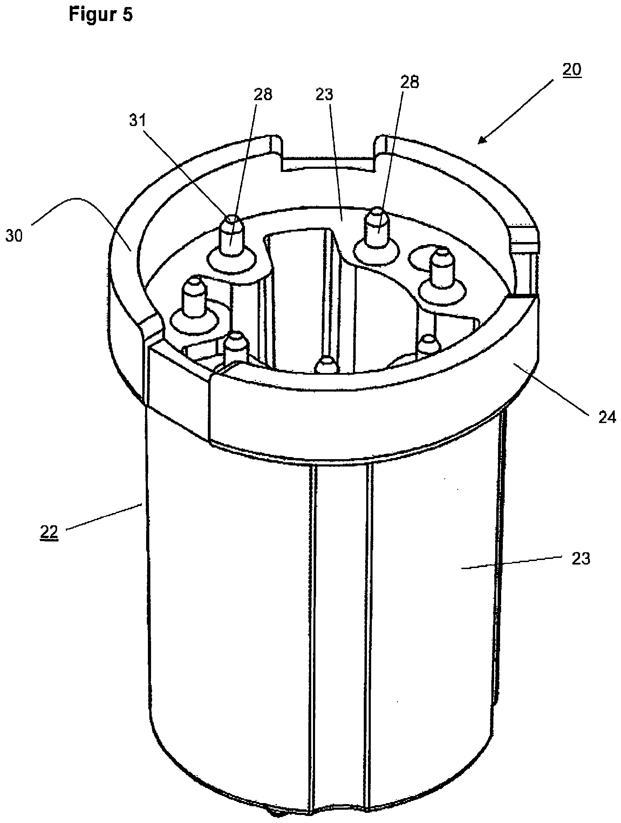

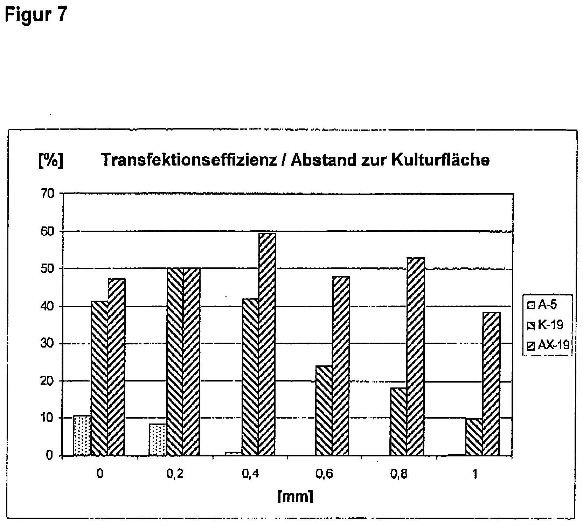

**Figur 3** zeigt eine perspektivische Ansicht der Unterseite einer beispielhaften Ausführungsform einer Elektrodenanordnung 20 einer erfindungsgemäßen Vorrichtung. Die Elektrodenanordnung 20 umfasst sieben Elektroden 21, welche im Folgenden in Bezug auf die Figuren 5 und 7 noch näher beschrieben sind. Die Elektroden 21 sind in einer Halterung 22 angeordnet, die im Wesentlichen zylinderförmig ausgebildet Ist. Die Halterung 22 umfasst einen Grundkörper 23 und einen am oberen Ende des Grundkörpers 23 angeordneten Randbereich 24, wobei der Außendurchmesser des Randbereichs 24 größer als der Außendurchmesser des Grundkörpers 23 ist, so dass der Randbereich 24 nach außen über den Grundkörper 23 hinausragt. Die Elektroden 21 sind zum größten Teil innerhalb des Grundkörpers 23 angeordnet und liegen mit ihrer unteren Stirnfläche 33 an der Unterseite 25 der Halterung 22 frei, so dass sie mit der Umgebung in Kontakt stehen. Die einzelnen Elektroden 21 sind jeweils durch ein isolierendes Material 26 elektrisch voneinander getrennt, wobei das isolierende Material 26 in diesem Ausführungsbeispiel den Raum zwischen den einzelnen Elektroden 21 vollständig ausfüllt. Das isolierende Material 26 zwischen den sich jeweils gegenüberliegenden Flächen der Elektroden 21 gewährleistet, dass beim Anlegen einer Spannung an die Elektroden kein Strom über den Raum zwischen den Elektroden 21 abfließen kann, wenn die Elektroden 21 in eine elektrisch leitende Flüssigkeit eingetaucht sind. Das isolierende Material 26 bewirkt vielmehr, dass beim Anlegen einer Spannung an die Elektroden 21 Strom über die Stirnflächen 33 der Elektroden 21 fließt und sich ein elektrisches Feld unterhalb der Unterseite 25 der Halterung 22 bildet. Da keine nennenswerten Ströme über den Raum zwischen den Elektroden 21 abfließen können, fällt die Spannung bei der Entladung eines Kondensators oder einer anderen nicht-permanenten Spannungsquelle nur langsam ab, so dass über die Zeit stabilere Ströme fließen, die ein für die meisten biologischen Verfahren, beispielsweise die Elektrotransfektion, ausreichend starkes elektrisches Feld über die Zeit der Entladung erzeugen. Die Elektrodenanordnung 20 ist insbesondere dafür vorgesehen, in ein zumindest teilweise mit Flüssigkeit gefülltes Gefäß, beispielsweise ein Reaktionsbehältnis, eine Zellkulturschale oder ein "Well" einer Multiwell-Platte, eingesetzt zu werden, wobei dieses Gefäß eine Bodenfläche aufweist, an der lebende Zellen anhaften können.

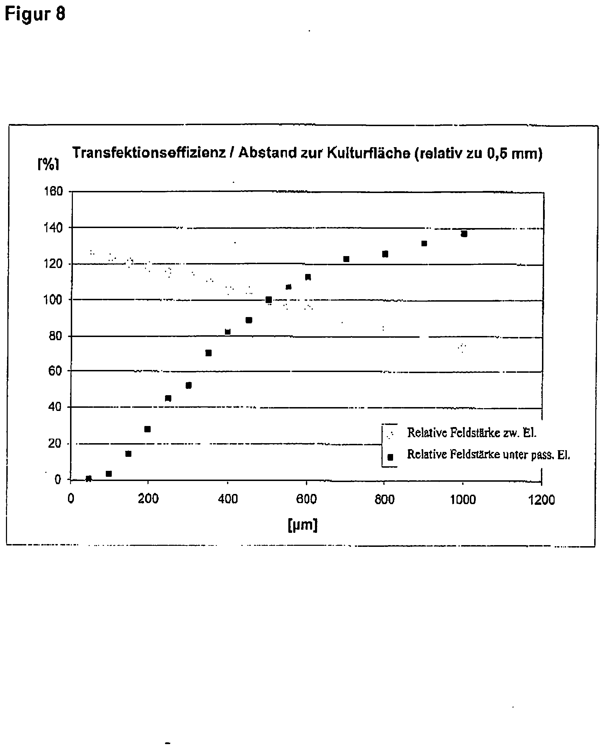

Die adhärenten Zellen auf der Bodenfläche des Gefäßes sind üblicherweise mit einer geeigneten Flüssigkeit, beispielsweise einem Zellkulturmedium oder einer an die gewünschte elektrische Behandlung adaptierten Lösung, bedeckt, wobei die Elektrodenanordnung 20 beim Einsetzen in das Gefäß zumindest einen Teil dieser Flüssigkeit verdrängt. Damit die Elektroden 21 mit ihren Stirnflächen 33 nicht direkt auf der Bodenfläche des Gefäßes und damit auf den Zellen aufliegen, weist die Unterseite 25 der Halterung 22 vier Abstandhalter 27 auf, die einen ausreichenden Abstand der Elektroden 21 zur Bodenfläche des Gefäßes gewährleisten. Durch das Einstellen des Abstands auf einen empirisch ermittelten Wert kann die Effizienz des erfindungsgemäßen Verfahrens optimiert werden (siehe Figuren 7 und 8).

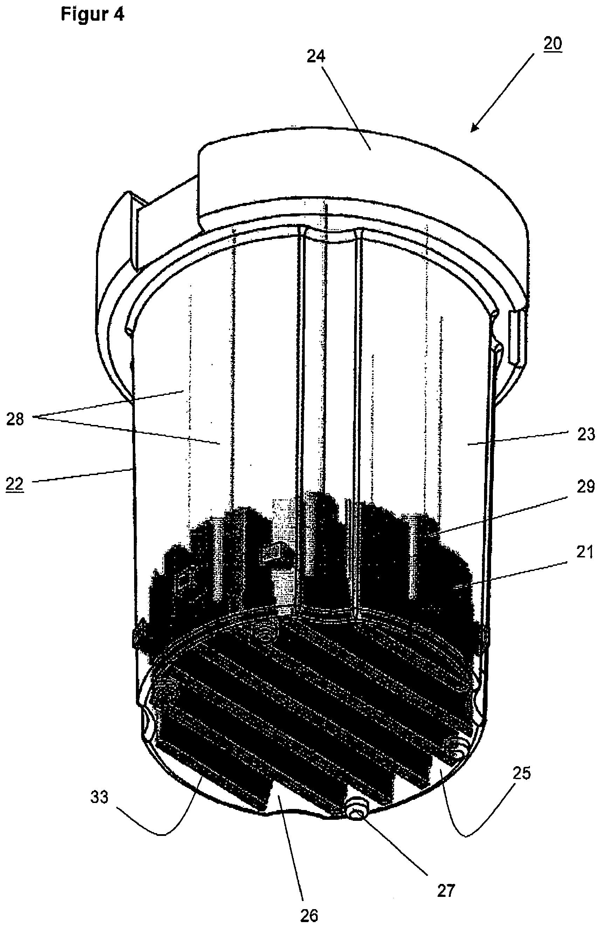

**Figur 4** zeigt eine perspektivische Ansicht der Elektrodenanordnung 20 gemäß Figur 3, wobei in dieser Darstellung die innen liegenden Teile der Elektroden 21 sichtbar dargestellt sind. Es wird in dieser Darstellung deutlich, dass die Elektroden 21 im Wesentlichen plattenförmig ausgebildet sind, wobei die Dicke der Elektrodenplatten sich in Richtung der Unterseite 25 der Halterung 22 verringert. Die freiliegenden Stirnflächen 33 der Elektroden 21, die mit der Flüssigkeit in dem Gefäß in Kontakt stehen, sind also wesentlich schmaler als die innerhalb des Grundkörpers 23 angeordneten Teile der Elektroden 21. Dies hat den Vorteil, dass der Bereich unterhalb der jeweiligen Elektrode 21, innerhalb dessen eine effektive elektrische Behandlung der Zellen aufgrund des zu schwachen elektrischen Feldes nicht möglich ist, verringert wird. Am gegenüberliegenden Ende müssen die Elektroden 21 dagegen eine größere Dicke aufweisen, da sie hier zur Herstellung eines ausreichenden elektrischen Kontakts effektiv kontaktiert werden müssen. Der elektrische Kontakt zu der jeweils verwendeten Spannungsquelle wird hierbei im vorliegenden Ausführungsbeispiel über stiftförmige Kontaktelemente 28 hergestellt, die in verdickte Bereiche 29 der Elektroden 21 eingesetzt sind. Die Kontaktelemente 28 werden jeweils an ihrem dem Bereich 29 gegenüberliegenden Ende mittels einer geeigneten Kontaktvorrichtung elektrisch mit einer Spannungsquelle verbunden. Bei der Spannungsquelle kann es sich beispielsweise um einen oder mehrere Kondensatoren handeln, der bzw. die die kontrollierte Abgabe von Spannungsimpulsen ermöglicht bzw. ermöglichen. Die erzeugten Spannungsimpulse werden über die Kontaktelemente 28 an die Elektroden 21 weitergeleitet, so dass an der Unterseite der Elektroden 21, d. h. unterhalb der Unterseite 25 der Halterung 22, ein elektrisches Feld entsteht, welches aufgrund des isolierenden Materials 26 zwischen den Elektroden 21 auf den Raum zwischen den Zellen und der den Zellen zugewandten Seite der Elektroden 21 begrenzt bzw. fokussiert ist.

Die erfindungsgemäße Elektrodenanordnung 20 wird vorzugsweise im Spritzgussverfahren hergestellt. Dabei werden zunächst die Kontaktelemente 28 in ein geeignetes Spritzgusswerkzeug eingelegt und dann mit einem elektrisch isolierenden Polymer umspritzt. In einem zweiten Schritt wird dann ein elektrisch leitendes Polymer eingespritzt, das die Elektroden 21 bildet. Alternativ können die Elektroden auch aus einem Metall, vorzugsweise Aluminium, bestehen. Bei dieser Ausführungsform werden zunächst die Metallelektroden in das Spritzgusswerkzeug eingelegt und dann von einem elektrisch isolierenden Polymer umspritzt. Bei dieser Ausführungsform weisen die Metallelektroden vorzugsweise nach oben hinausragende Fortsätze auf, über welche die Elektroden elektrisch kontaktiert werden können.

**Figur 5** zeigt eine perspektivische Ansicht der Oberseite 30 der erfindungsgemäßen Elektrodenanordnung 20 gemäß Figur 3. Es wird hier deutlich, dass die Kontaktelemente 28 nach oben aus dem Grundkörper 23 herausragen. Die Kontaktelemente 28 sind also bis auf die freiliegenden Enden 31 und den gegenüber liegenden in das elektrisch leitende Polymer eintauchenden Enden vollständig von dem elektrisch isolierenden Material des Grundkörpers 23 umgeben. Über die freiliegenden Enden 31 können die Kontaktelemente 28 mittels einer geeigneten Vorrichtung elektrisch mit einer Spannungsquelle verbunden werden.

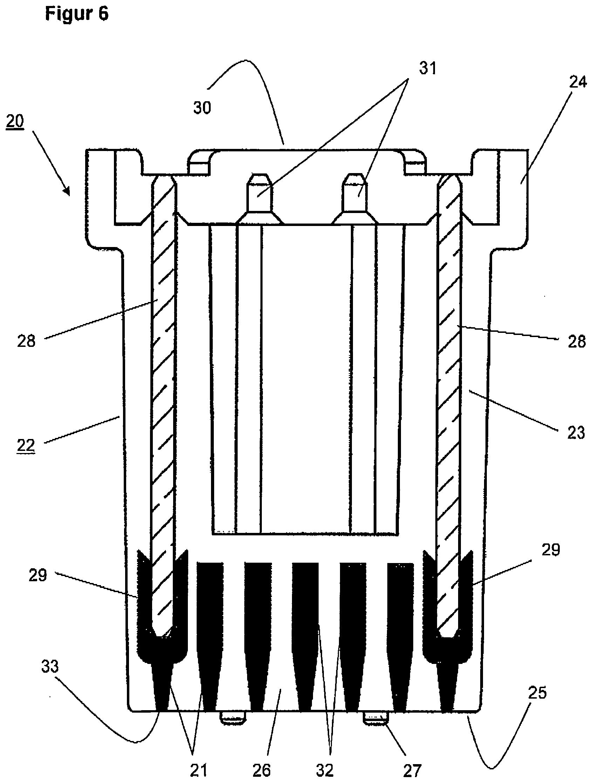

**Figur 6** zeigt einen Längsschnitt durch die Elektrodenanordnung 20 gemäß den Figuren 3 bis 5. In dieser Darstellung wird deutlich, dass sich der Durchmesser der Elektroden 21 in Richtung der Unterseite 25 des Grundkörpers 23 verjüngt, so dass die Fläche 33 unterhalb der Elektroden 21, innerhalb der sich nur ein unzureichendes elektrisches Feld ausbildet, verringert wird. Am entgegengesetzten Ende der Elektroden 21 befindet sich der Bereich 29 mit vergrößerter Dicke, in den jeweils die Kontaktelemente 28 eingesetzt bzw. eingespritzt sind. Diese besonders vorteilhafte Ausgestaltung gewährleistet einen ausreichenden elektrischen Kontakt zwischen den Kontaktelementen 28 und den Elektroden 21, so dass eine effektive Weiterleitung der Spannungsimpulse von der Spannungsquelle bis zu den Elektroden 21 sichergestellt ist. Wenn die Elektrodenanordnung 20 in ein mit Flüssigkeit gefülltes Gefäß, an dessen Bodenfläche lebende Zellen anhaften, eingesetzt wird, sorgen die Abstandhalter 27 dafür, dass ein optimaler Abstand zwischen der Unterseite der Elektroden 21 und den zu behandelnden Zellen eingestellt wird. Da der Raum zwischen den einander jeweils gegenüber liegenden Flächen 32 der Elektroden 21 mit dem isolierenden Material 26 vollständig ausgefüllt ist, gelangt keine Flüssigkeit zwischen die Flächen 32 der Elektroden 21, so dass kein Strom über den Bereich zwischen den Flächen 32 der Elektroden 21 abfließen kann. Auf diese Weise wird beim Anlegen einer Spannung an die Elektroden 21 das elektrische Feld an der den Zellen zugewandten Seite der Elektroden 21 konzentriert und auf den Raum zwischen den Zellen und den Elektroden 21 begrenzt bzw. fokussiert. Auf diese Weise können die Zellen sehr effektiv und unter relativ geringem Energieaufwand behandelt werden. Ein weiterer Vorteil der Erfindung liegt darin, dass die Elektrodenanordnung 20 beim Einsetzen in das Gefäß einen Teil der Flüssigkeit verdrängt, da keine Zwischenräume zwischen den Elektroden 21 vorhanden sind. Aus diesem Grund muss das Gefäß nur mit einer geringen Menge Flüssigkeit gefüllt sein, wodurch für die Behandlung benötigte Lösungen und Substanzen eingespart und somit Kosten reduziert werden können.

**Figur 7** zeigt die Abhängigkeit der Transfektionseffizienz vom Abstand der Elektroden zu den zu behandelnden Zellen jeweils mit unterschiedlich starken Spannungsimpulsen. Transfektion bedeutet in diesem Zusammenhang das Einbringen von Nukleinsäuremolekülen (hier DNA) in lebende (hier tierische) Zellen mittels elektrischer Spannungsimpulse. Während bei relativ hohen Spannungen (AX-19) nur eine geringe Abhängigkeit der Transfektionseffizienz vom Abstand zwischen den Elektroden und den Zellen vorliegt, zeigt sich bei schwachen Spannungsimpulsen (A-5), dass die Transfektionseffizienz um so größer wird, je geringer der Abstand zwischen den Elektroden und den Zellen ist. Mittelstarke Spannungsimpulse (K-19) zeigen dagegen ein Optimum bei mittelgroßen Abständen. Es wird also deutlich, dass der Abstand zwischen den Elektroden und den Zellen einen in Abhängigkeit von der Stärke der Spannungsimpulse mehr oder weniger großen Einfluss auf die Transfektionseffizienz hat. Um die Transfektionseffizienz zu optimieren, wird daher der Abstand erfindungsgemäß auf einen empirisch ermittelten Wert eingestellt, beispielsweise durch entsprechende Dimensionierung der Abstandhalter 27 der Elektrodenanordnung 20 gemäß den Figuren 3 bis 6.

**Figur 8** zeigt die relativen elektrischen Feldstärken im Bereich zwischen den Elektroden und dem Boden eines Gefäßes in Abhängigkeit vom Abstand der Elektroden zum Boden des Gefäßes. Die Feldstärke Ist dabei jeweils auf die Feldstärke bei einem Abstand von 500 µm bezogen, d. h. die Feldstärke beider Kurven wurde für diesen Abstand gleich 100 % gesetzt. Die Feldstärken zwischen den aktiven Elektroden und unterhalb der passiven Elektroden verhalten sich entgegengesetzt, d. h. während die Feldstärke zwischen den aktiven Elektroden mit kleiner werdenden Abständen größer wird, geht die Feldstärke unterhalb der passiven Elektroden gegen Null. Mit größer werdenden Abständen nimmt die Feldstärke zwischen den aktiven Elektroden ab, während die Feldstärke unterhalb der passiven Elektroden weiter zunimmt. Absolut gesehen nimmt die Feldstärke zwischen den aktiven Elektroden mit größer werdendem Abstand ab, während die Feldstärke unterhalb der potentialfreien Elektroden bei einem mittleren Abstand einen Maximalwert erreicht, also ausgehend von diesem Maximalwert bei kleiner oder größer werdenden Abständen abnimmt. Der Abstand der Elektroden zu den Zellen wird daher erfindungsgemäß in Abhängigkeit von den Reaktionsbedingungen derart eingestellt, dass die Feldstärke unterhalb der potentialfreien Elektroden dem Maximalwert möglichst nahe kommt und gleichzeitig zwischen den aktiven Elektroden einen möglichst hohen Wert erreicht.

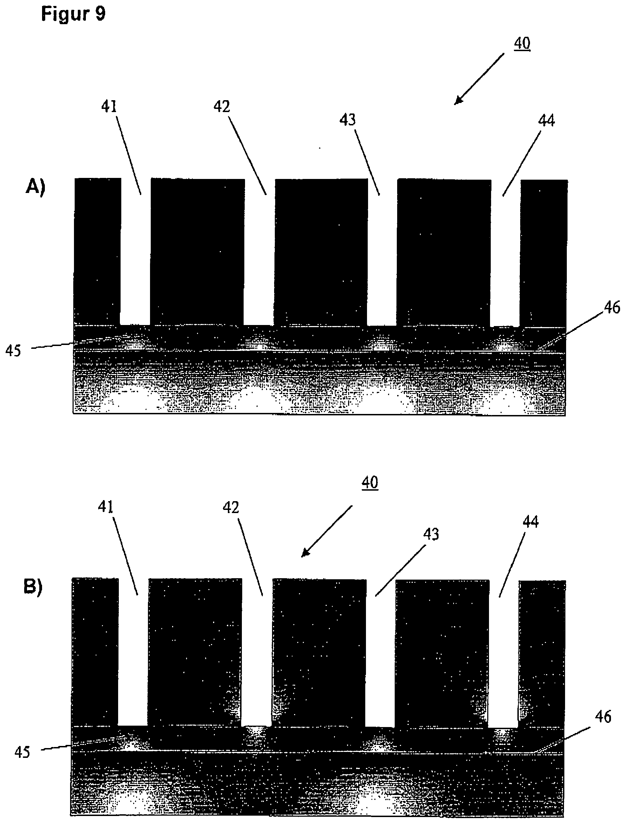

**Figur 9** zeigt Simulationen der elektrischen Felder in einem Zellkulturgefäß bei ausschließlich aktiven Elektroden (oben) sowie bei aktiven und potentialfreien Elektroden (unten). Während die bekannten Verfahren und Vorrichtungen zur Erzeugung elektrischer Felder den Nachteil haben, dass das elektrische Feld orthogonal unterhalb der aktiven Elektroden minimal und daher die elektrische Behandlung von Zellen in diesem Bereich unzureichend ist, wird hier deutlich, dass durch das Einbeziehen potentialfreier (passiver) Elektroden in das erfindungsgemäße Verfahren eine gleichmäßigere und somit effektivere Behandlung der Zellen ermöglicht wird. Die hier beispielhaft dargestellte Elektrodenanordnung 40 umfasst vier Elektroden 41, 42, 43, 44, die beim Anlegen einer elektrischen Spannung im Raum 45 zwischen den Elektroden 41, 42, 43, 44 und der Bodenfläche 46 eines Zellkulturgefäßes ein elektrisches Feld erzeugen. Die Stärke dieses elektrischen Feldes ist in dieser Darstellung durch die Dichte der Feldlinien simuliert. Dabei weisen die dunkleren Bereiche auf eine höhere Feldstärke und die helleren Bereiche auf eine geringere Feldstärke hin. In Abbildung A) sind alle Elektroden 41, 42, 43, 44 aktiv. In diesem Fall ist die Feldstärke lediglich zwischen den Elektroden 41, 42, 43, 44 und an deren freien Enden hoch (dunkle Bereiche). Dagegen ist die Feldstärke im Raum 45 in den Bereichen unterhalb der Elektroden 41, 42, 43, 44 gering (helle Bereiche), so dass die zu behandelnden Zellen auf der Bodenfläche 46 nur ungleichmäßig mit einem ausreichenden elektrischen Feld beaufschlagt werden. Erfindungsgemäß wird daher, wie in Abbildung B) dargestellt, in diesem Ausführungsbeispiel zunächst nur an die beiden Elektroden 41 und 43 Spannung angelegt, während die anderen beiden Elektroden 42 und 44 potentialfrei (passiv) sind. Dies hat zur Folge, dass in den Bereichen unterhalb der passiven Elektroden 42 und 44 ein stärkeres, nur gering durch die passiven Elektroden 42 und 44 abgeschwächtes elektrisches Feld auf die auf der Bodenfläche 46 anhaftenden Zellen wirkt. Wenn nun bei einem weiteren Spannungsimpuls an die zuvor passiven Elektroden 42 und 44 Spannung angelegt wird und die zuvor aktiven Elektroden 41 und 43 potentialfrei sind, wirkt auch im Bereich unterhalb der Elektroden 41 und 43 ein stärkeres elektrisches Feld. Folglich können auf der Bodenfläche 46 anhaftenden Zellen mittels des erfindungsgemäßen Verfahrens gleichmäßig mit einen ausreichenden elektrischen Feld behandelt werden.

**Figur 10** zeigt mikroskopische Bilder von fluoreszierend markierten Zellen, die gemäß dem Stand der Technik (A) und mittels des erfindungsgemäßen Verfahrens (B und C) transfiziert wurden. Bei der Elektrotransfektion der in Abbildung A) dargestellten Zellen waren alle Elektroden aktiv, d. h. alle Elektroden waren entweder mit Hochspannung oder mit Masse verbunden. Die erfolgreich mit dem Plasmid transfizierten Zellen fluoreszieren und sind somit deutlich zu identifizieren. Es zeigen sich in Abbildung A) aber Lücken ("Schatten") im Rasen der transfizierten Zellen, die den Bereichen unterhalb der aktiven Elektroden entsprechen. In diesen Bereichen war das elektrische Feld nicht ausreichend stark, um die Zellen erfolgreich zu transfizieren. Bei der Elektrotransfektion der in Abbildung B) dargestellten Zellen war nur die Hälfte der Elektroden aktiv, während die andere Hälfte potentialfrei war. Im Vergleich zur Transfektion gemäß Abbildung A) sind hier insgesamt weniger Zellen erfolgreich transfiziert worden, es sind aber auch weniger Lücken vorhanden bzw. die Abstände zwischen den Bereichen mit sehr geringer Transfektionseffizienz sind größer. Die Transfektionseffizienz ist hier zunächst geringer, da das elektrische Feld über die gesamte Kulturfläche aufgrund der größeren Anstände zwischen den aktiven Elektroden insgesamt schwächer ist. Gemäß dem erfindungsgemäßen Verfahren wurden dieselben Zellen daraufhin nochmals mit einem zweiten elektrischen Feld (Spannungsimpuls) beaufschlagt, wobei beim zweiten Impuls aktive und passive Elektroden vertauscht waren, d. h. alle beim erstem Impuls aktiven Elektroden waren beim zweiten Impuls potentialfrei, während die zuvor passiven Elektroden beim zweiten Impuls aktiv waren. Es sind jetzt keine Lücken mehr zu erkennen, d. h. alle Bereiche, einschließlich der Bereiche unterhalb der Elektroden, zeigen eine hohe Transfektionsrate (Abbildung C). Das erfindungsgemäße Verfahren ermöglicht also eine über die gesamte Kulturfläche sehr gleichmäßige und homogene Behandlung der Zellen. Insbesondere der Vergleich der Abbildungen A) (Stand der Technik) und C) (erfindungsgemäßes Verfahren) veranschaulicht dabei die deutlichen Vorteile des erfindungsgemäßen Verfahrens gegenüber den bekannten Verfahren.

**Figur 11** zeigt mikroskopische Bilder von fluoreszierenden Zellen, die gemäß dem erfindungsgemäßen Verfahren transfiziert wurden, wobei der Abstand zwischen zwei Spannungsimpulsen variiert wurde. Bei sehr geringen Abständen (A: < 0,5 Sekunden) kommt es zu einer unerwünschten Verringerung der Transfektionseffizienz. Dieser Effekt wird mit größer werdenden Abständen zwischen den Spannungsimpulsen wieder aufgehoben (B: 2,0 Sekunden). Damit die gesamte Prozessdauer nicht unnötig in die Länge gezogen wird, sollten die Abstände optimiert, d. h. an die Prozessbedingungen, wie beispielsweise elektrische Parameter, Probenvolumen, Pufferlösung bzw. Kulturmedium, Temperatur und/oder Zelltyp, angepasst werden. Vorzugsweise wird der zeitliche Abstand zwischen zwei Spannungsimpulsen genutzt, um zunächst mehrere Proben nacheinander mit dem ersten Spannungsimpuls und dann diese Proben nacheinander mit dem zweiten Spannungsimpuls zu beaufschlagen. Durch diese besonders vorteilhafte zeitliche Verschachtelung der Spannungsimpulse kann die gesamte Prozessdauer bei der Behandlung mehrerer Proben minimiert werden.

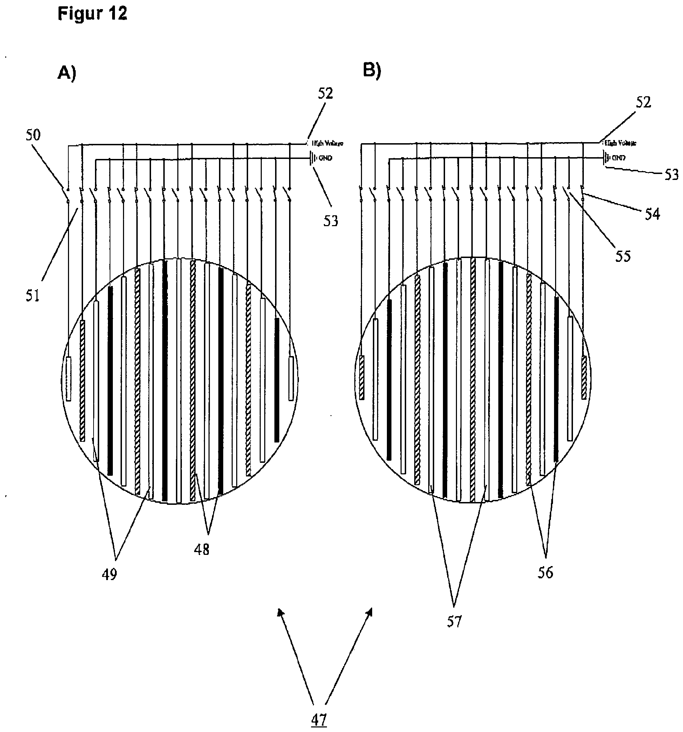

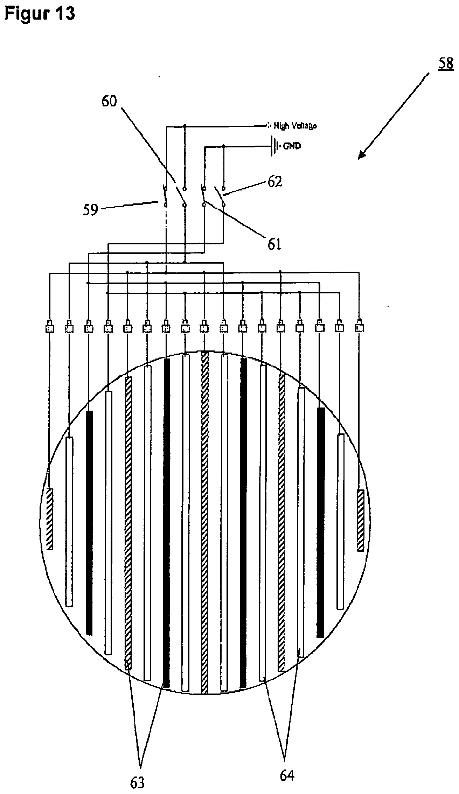

Zur Durchführung des erfindungsgemäßen Verfahrens müssen die Elektroden entweder individuell, wie In Figur 12 gezeigt, oder in Gruppen, wie in Figur 13 gezeigt, adressiert werden. Dies erfolgt vorzugsweise über Schaltvorrichtungen, beispielsweise IGBTs, MOSFETs oder Relais, welche einen offenen oder einen geschlossenen Zustand einnehmen können. Im offenen Zustand leiten die Schaltvorrichtungen keinen elektrischen Strom, so dass die angeschlossenen Elektroden elektrisch isoliert und somit potentialfrei (passiv) sind. In geschlossenem Zustand leiten die Schaltvorrichtungen elektrischen Strom, so dass Strom durch die angeschlossenen Elektroden fließen kann. Diese Elektroden haben ein elektrisches Potential (Plus oder Minus) und wirken als Anode oder Kathode. Sie werden als aktive Elektroden bezeichnet.

**Figur 12** zeigt eine Schaltungsanordnung 47 zur Durchführung des erfindungsgemäßen Verfahrens mit siebzehn Elektroden 48, 49, 56, 57. Die planparallel angeordneten Elektroden 48, 49, 56, 57 sind jeweils über eine Schaltvorrichtung 50, 51, 54, 55 entweder mit einer Spannungsquelle 52 oder Masse 53 verbunden. Abbildung A) zeigt den Zustand der Schaltungsanordnung 47 beim Erzeugen des ersten elektrischen Feldes. Der Teil der Elektroden 48, 49, die mit den offenen Schaltvorrichtungen 50 verbunden sind, sind hier zunächst potentialfreie Elektroden 49. Der Teil der Elektroden 48, 49, die mit den geschlossenen Schaltvorrichtungen 51 verbunden sind, sind die aktiven Elektroden 48, welche letztendlich das erste elektrische Feld zur Behandlung der Zellen erzeugen. Orthogonal unterhalb der aktiven Elektroden 48 ist das elektrische Feld minimal, so dass die Zellen in diesen Bereichen nicht ausreichend behandelt werden. Abbildung B) zeigt den Zustand der Schaltungsanordnung 47 beim Erzeugen des zweiten elektrischen Feldes. Die Schaltvorrichtungen 54, die beim Erzeugen des ersten elektrischen Feldes offen waren, sind hier geschlossen, während die Schaltvorrichtungen 55, die beim Erzeugen des ersten elektrischen Feldes geschlossen waren, hier offen sind. Dies hat zur Folge, dass die Elektroden 56, die beim Erzeugen des ersten elektrischen Feldes potentialfrei waren, beim Erzeugen des zweiten elektrischen Feldes die aktiven Elektroden sind, welche das zweite elektrische Feld erzeugen. Die Elektroden 57, die beim Erzeugen des ersten elektrischen Feldes aktiv waren, sind beim Erzeugen des zweiten elektrischen Feldes potentialfrei. Da die aktiven Elektroden 56 beim Erzeugen des ersten elektrischen Feldes alle zwischen zwei potentialfreien Elektroden 57 angeordnet sind, werden die Bereiche unterhalb der aktiven Elektroden 56 von dem zweiten elektrischen Feld beaufschlagt, das durch die aktiven Elektroden 56 erzeugt wird. Folglich werden durch das zweite elektrische Feld auch die Zellen elektrisch behandelt, die während der Dauer des ersten elektrischen Feldes nicht ausreichend behandelt wurden, so dass insgesamt alle Zellen gleichmäßig behandelt werden.

**Figur 13** zeigt eine beispielhafte erfindungsgemäße Vorrichtung 58 zur Durchführung des erfindungsgemäßen Verfahrens mit vier Schaltvorrichtungen 59, 60, 61, 62. Die Schaltvorrichtungen 59 und 60 sind an eine Spannungsquelle angeschlossen, während die Schaltvorrichtungen 61 und 62 mit Masse verbunden sind. Die Elektroden (aktive Elektroden 63: gestrichelt oder schwarz, potentialfreie Elektroden 64: farblos) der Vorrichtung 58 sind in zwei Gruppen aufgeteilt, wobei eine Gruppe mit den Schaltvorrichtungen 59 und 61 und die andere Gruppe mit den Schaltvorrichtungen 60 und 62 elektrisch gekoppelt sind. Bei der hier dargestellten Ausführungsform sind also siebzehn Elektroden 63, 64 über lediglich vier Schaltvorrichtungen 59, 60, 61, 62 mit einer Spannungsquelle verbunden. In dem hier dargestellten Zustand sind die aktiven Elektroden 63 (erste Gruppe) über die geschlossenen Schaltvorrichtungen 59 und 61 mit einer Spannungsquelle bzw. Masse verbunden, so dass beim Anlegen einer Spannung an die aktiven Elektroden 63 ein erstes elektrisches Feld zwischen den aktiven Elektroden 63 entsteht. Da die Schaltvorrichtungen 60 und 62 offen sind, bleiben die hiermit gekoppelten Elektroden 64 (zweite Gruppe) in diesem Zustand potentialfrei bzw. passiv. Um auch den Zellen in dem Bereich unterhalb der aktiven Elektroden 63, die im hier dargestellten Zustand nur einem minimalen elektrischen Feld ausgesetzt waren, eine ausreichende Behandlung zukommen zu lassen, werden erfindungsgemäß zur Erzeugung eines zweiten elektrischen Feldes die Schaltvorrichtungen 59 und 61 geöffnet und die Schaltvorrichtungen 60 und 62 geschlossen. Durch diesen einfachen Schaltvorgang werden die Elektroden der ersten Gruppe, die im hier dargestellten Zustand aktive Elektroden 63 sind, zu potentialfreien Elektroden, während die Elektroden der zweiten Gruppe, die im hier dargestellten Zustand potentialfreie Elektroden 64 sind, aktiv werden und ein zweites elektrisches Feld erzeugen. Dieses zweite elektrische Feld sorgt dann dafür, dass auch die Bereiche unterhalb der Elektroden der ersten Gruppe 63 eine ausreichende elektrische Behandlung erfahren, so dass insgesamt eine gleichmäßige und effiziente Behandlung der Zellen gewährleistet ist. Die erfindungsgemäße Vorrichtung 58 hat dabei den Vorteil, dass sie mit vier Schaltvorrichtungen auskommt und durch Schalten dieser Schaltvorrichtungen schnell zwischen den Elektroden beider Gruppen umschalten kann. Jede Gruppe von Elektroden kann dabei entweder aktiv oder passiv geschaltet werden, wobei alle Elektroden einer Gruppe zur gleichen Zeit immer gleich geschaltete sind (aktiv oder potentialfrei). Durch die Gruppierung der Elektroden 63, 64 weist die erfindungsgemäße Vorrichtung 58 also eine beispielsweise im Vergleich zu der Schaltungsanordnung 47 gemäß Figur 12 deutlich reduzierte Anzahl von Schaltvorrichtungen auf, was den apparativen Aufwand und die Störanfälligkeit der Vorrichtung 58 signifikant reduziert.

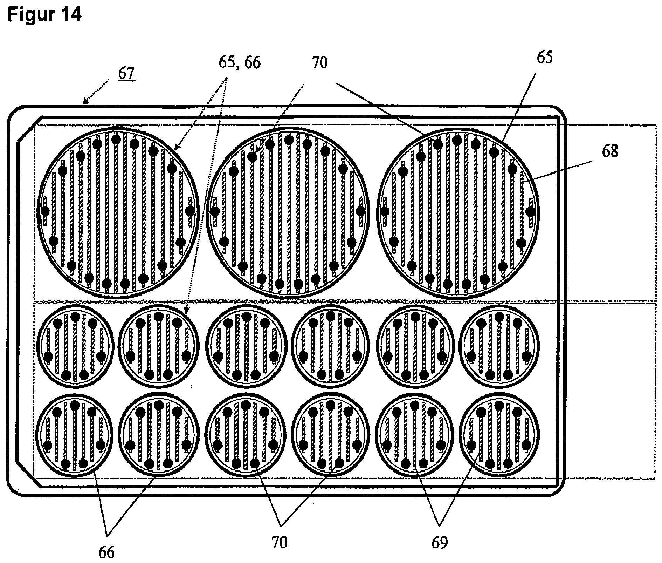

**Figur 14** zeigt schematische Darstellungen von Elektrodenanordnungen 65, 66 mit unterschiedlichen Abmessungen für Zellkulturplatten mit 3 größeren Kulturräumen ("6-well"-Format, Elektrodenanordnungen 65) und 12 kleineren Kulturräumen ("24-well"-Format, Elektrodenanordnungen 66). Um Material einzusparen und den konstruktiven Aufwand zu verringern, werden erfindungsgemäß gemeinsame Kontaktpunkte für Elektrodenanordnungen mit unterschiedlichen Abmessungen ausgewählt. Hierdurch kann die Anzahl der Kontaktelemente, die geräteseitig zum elektrischen Kontaktieren der Elektroden benötigt werden, in vorteilhafter Weise signifikant verringert werden. Der hier dargestellte Aufnahmebereich 67 für Zellkulturplatten weist eine Sektion für drei größere Kulturräume und eine Sektion für zwölf kleinere Kulturräume auf. Die größeren Kulturräume werden zur elektrischen Behandlung der darin befindlichen Zellen mit entsprechend dimensionierten Elektrodenanordnungen 65 bestückt, beispielsweise in Form von Eintauchelektroden, die jeweils siebzehn Elektroden 68 umfassen. Die kleineren Kulturräume werden zur elektrischen Behandlung der darin befindlichen Zellen mit entsprechend dimensionierten Elektrodenanordnungen 66 bestückt, beispielsweise ebenfalls in Form von Eintauchelektroden, die jeweils sieben Elektroden 69 umfassen. Die Elektrodenanordnungen können beispielsweise in einen Deckel für Zellkulturplatten integriert oder an einer Trägereinheit der entsprechenden Kontaktierungsvorrichtung angebracht sein. Eine solche Kontaktierungsvorrichtung umfasst auch die Kontaktelemente, die in der jeweiligen Sektion des Aufnahmebereichs 67 wie nachstehend erläutert entsprechend der Kontaktpunkte der Elektroden angeordnet sind. Jeder Elektrode 68, 69 ist jeweils ein Kontaktpunkt 70 zugeordnet, an dem die jeweilige Elektrode 68, 69 mittel jeweils eines Kontaktelements kontaktiert werden kann, damit ein elektrischer Kontakt zu einer Spannungsquelle, beispielsweise einem Kondensator, hergestellt werden kann. Eine Vorrichtung zur Kontaktierung einer solchen "gemischten" Zellkulturplatte 67 benötigt also 3 x 17 + 12 x 7 = 135 Kontaktelemente. Eine Vorrichtung zur Kontaktierung von zwei unterschiedlichen Formaten von Zellkulturplatten, beispielsweise "6-well"-Format (6 Kulturräume für Elektrodenanordnungen mit jeweils 17 Elektroden) und "24-well"-Format (24 Kulturräume mit jeweils 7 Elektroden), würde sogar 6 x 17 + 24 x 7 = 270 Kontaktelemente benötigen.

Da zur Herstellung eines ausreichenden elektrischen Kontakts durch beispielsweise federbeaufschlagte Kontaktelemente ein gewisser Anpressdruck, der auch Herstellungstoleranzen ausgleicht, erforderlich ist, sollte die Anzahl an Kontaktpunkten bzw. entsprechenden Kontaktelementen möglichst gering sein. So beträgt die Anpresskraft einer Standardfeder etwa 0,4 N, was bei einer Anzahl von 270 Kontaktelementen insgesamt zu einer mechanischen Kraft von ungefähr 108 N (ca. 10 Kg) führen würde. Eine Minimierung der Anzahl an Kontaktelementen ist daher zu Verringerung des technischen und apparativen Aufwands erstrebenswert.

**Figur 15** zeigt beispielhafte und bevorzugte Anordnungen der Kontaktpunkte 71, 72, 73 für Elektrodenanordnungen 74, 75 mit unterschiedlichen Abmessungen für Zellkulturplatten mit mindestens einem größeren Reaktionsraum (Elektrodenanordnung 74) und mindestens vier kleineren Reaktionsräumen (Elektrodenanordnungen 75). Die meisten Kontaktpunkte 71, 72, 73 können erfindungsgemäß derart angeordnet werden, dass sie für die unterschiedlichen Formate Identisch sind, also als Kontaktpunkte sowohl für Elektroden der größeren Elektrodenanordnung 74 als auch für Elektroden der kleineren Elektrodenanordnungen 75 dienen können. Bei den hier beispielhaft dargestellten Formaten nehmen lediglich die mittleren Elektroden (Raster-Nm. 8-10) und die äußersten Elektroden (Raster-Nm. 1 und 17) der größeren Elektrodenanordnung 74 jeweils eine Position ein, die sich nicht mit entsprechenden Positionen von Elektroden der kleineren Elektrodenanordnungen 75 in Übereinstimmung bringen lässt. Die übrigen Kontaktpunkte (Raster-Nm. 2-7 und 11-16) können jeweils als Kontaktpunkte für die entsprechenden Elektroden der Elektrodenanordnungen beider Formate verwendet werden (Abbildung A). Den hierbei noch nicht erfassten Elektroden der kleineren Elektrodenanordnungen 75 müssen dann jeweils eigene Kontaktpunkte zugeordnet werden. Eine erfindungsgemäße Anordnung der Kontaktpunkte 72, 73, die auch im Hinblick auf den Produktionsprozess optimiert ist, ist in Abbildung B) dargestellt. Die siebzehn schwarzen Kontaktpunkte 72 repräsentieren Stellen, an denen alle Elektroden der größeren Elektrodenanordnung 74 (z.B. "6-well-Format) und einige Elektroden der kleineren Elektrodenanordnungen 75 (z.B. "24-well-Format") kontaktiert werden können. Die sechzehn schraffierten Kontaktpunkte 73 repräsentieren Stellen, an denen ausschließlich die verbleibenden Elektroden der kleineren Elektrodenanordnungen 75 kontaktiert werden können. Insgesamt käme eine Vorrichtung zur Kontaktierung dieser beiden Formate bei entsprechender Anordnung der Kontaktelemente mit 6 x (17 + 16) = 198 Kontaktelementen innerhalb eines Aufnahmebereichs für Zellkulturplatten aus. Dies ist eine im Vergleich zu dem in Figur 14 gezeigten Beispiel (270 Kontaktelemente) deutlich reduzierte Anzahl an Kontaktelementen, was den apparativen Aufwand erheblich reduziert.

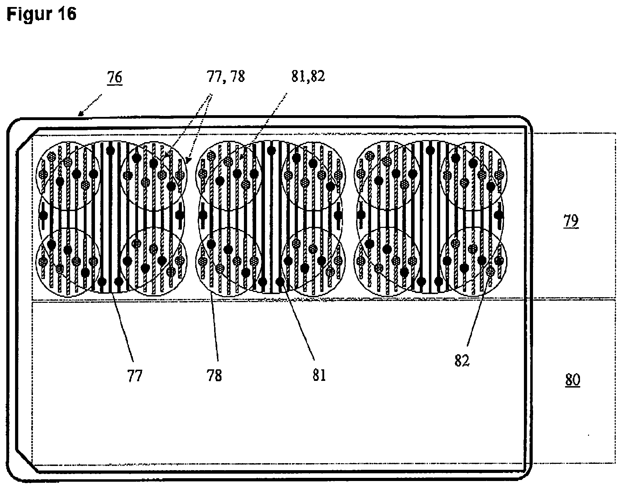

**Figur 16** zeigt eine weitere beispielhafte und bevorzugte Anordnung der Kontaktpunkte für Elektrodenanordnungen mit unterschiedlichen Abmessungen für Zellkulturplatten mit 3 größeren Reaktionsräumen ("6-well"-Format) und 12 kleineren Reaktionsräumen ("24-well"-Format). Bei dieser besonders vorteilhaften Ausführungsform sind die Elektroden und/oder die entsprechenden Kontaktelemente zur Kontaktierung der Kontaktpunkte um eine Achse drehbar, vorzugsweise eine senkrecht zur Ebene der Zellkulturplatte angeordnete Achse. Hierdurch kann die Anzahl der geräteseitig erforderlichen Kontaktelemente in vorteilhafter Weise weiter auf 3 x (17 + 16) = 99 Kontaktelemente reduziert werden. Darüber hinaus ermöglicht diese Ausführungsform die zusätzliche Behandlung von Zellkulturplatten anderer Formate, beispielsweise im "12- und 48-well" - Format). Im hier dargestellten Ausführungsbeispiel ist ein Aufnahmebereich 76 für Zellkulturplatten vorgesehen, der beispielsweise Teil einer Vorrichtung zur Kontaktierung von Elektrodenanordnungen 77, 78 sein kann. Diese Vorrichtung weist hier nicht dargestellte Kontaktelemente auf, die zu den Elektroden der Elektrodenanordnungen 77, 78 einen elektrischen Kontakt herstellen können. Der Aufnahmebereich 76 umfasst zwei Sektionen 79, 80, innerhalb derer jeweils Kontaktelemente in einer der Kontaktpunkte 81, 82 der Elektrodenanordnungen 77, 78 entsprechenden Anordnung vorgesehen sind. Die schwarzen Kontaktpunkte 81 repräsentieren Stellen, an denen alle Elektroden der größeren Elektrodenanordnungen 77 ("6-well-Format") und einige Elektroden der kleineren Elektrodenanordnungen 78 ("24-well-Format") kontaktiert werden können. Die schraffierten Kontaktpunkte 82 repräsentieren Stellen, an denen ausschließlich die verbleibenden Elektroden der kleineren Elektrodenanordnungen 78 kontaktiert werden können. Die erste Sektion 79 des Aufnahmebereichs 76 ist für die Elektrodenanordnungen 77, 78 bzw. entsprechend dimensionierte Zellkulturräume ("wells") vorgesehen, wobei in diesem Ausführungsbeispiel größere Elektrodenanordnungen 77 ("6-well"-Format) und kleinere Elektrodenanordnungen 78 ("24-well"-Format) elektrisch kontaktiert werden können. Wird also in den Aufnahmebereich 76 eine Zellkulturplatte mit 6 oder 24 Kulturräumen oder eine gemischte Platte mit 3 größeren und 12 kleineren Kulturräumen eingesetzt, so kann in der ersten Sektion 79 zunächst eine Hälfte der entsprechenden Elektrodenanordnungen elektrisch kontaktiert werden. Nach erfolgter Behandlung der Zellen in den Kulturräumen wird dann die Platte horizontal um 180° gedreht, so dass die andere Hälfte der Kulturräume in der ersten Sektion 79 angeordnet ist und elektrisch kontaktiert werden kann. Alternativ kann anstelle der Platte selbstverständlich auch die Einrichtung mit den Kontaktelementen gedreht werden, um die jeweils andere Hälfte der Platte zu behandeln. Die zweite Sektion 80 des Aufnahmebereichs 76 ist entweder frei und bleibt ungenutzt, oder ist für die Elektrodenanordnungen bzw. entsprechend dimensionierte Zellkulturräume ("wells") mit anderen Abmessungen vorgesehen, beispielsweise größere Elektrodenanordnungen im "2-well"-Format und kleinere Elektrodenanordnungen im "48-well"-Format. Mit einer erfindungsgemäßen Vorrichtung zur Kontaktierung von mindestens zwei Elektrodenanordnungen können also in vorteilhafter Weise mindestens zwei, vorzugsweise vier, unterschiedliche Plattenformate elektrisch kontaktiert werden.

### Bezugszeichenliste:

- 1: Elektrodenanordnung
- 2: Elektrode
- 3: Elektrode
- 4: Elektrode
- 5: Isolierendes Material
- 6: Innenraum
- 7: Gefäß
- 8: Bodenfläche
- 9: Flächen
- 10: Elektrodenanordnung
- 11: Elektrode
- 12: Elektrode
- 13: Elektrode
- 14: Elektrode
- 15: Isolierendes Material
- 16: Innenraum
- 17: Gefäß
- 18: Bodenfläche
- 20: Elektrodenanordnung
- 21: Elektroden
- 22: Halterung
- 23: Grundkörper
- 24: Randbereich
- 25: Unterseite
- 26: Isolierendes Material
- 27: Abstandhalter
- 28: Kontaktelemente
- 29: Bereich
- 30: Oberseite
- 31: Ende
- 32: Fläche
- 33: Stirnfläche
- 40: Elektrodenanordnung
- 41: Elektrode
- 42: Elektrode
- 43: Elektrode
- 44: Elektrode
- 45: Raum
- 46: Bodenfläche
- 47: Elektrodenanordnung
- 48: Aktive Elektroden
- 49: Potentialfreie Elektroden
- 50: Offene Schaltvorrichtungen
- 51: Geschlossene Schaltvorrichtungen
- 62: Hochspannung
- 53: Masse
- 54: Geschlossene Schaltvorrichtungen
- 55: Offene Schaltvorrichtungen
- 56: Aktive Elektroden
- 57: Potentialfreie Elektroden
- 58: Vorrichtung
- 59: Schaltvorrichtung
- 60: Schaltvorrichtung
- 61: Schaltvorrichtung
- 62: Schaltvorrichtung
- 63: Aktive Elektroden
- 64: Potentialfreie Elektroden
- 65: Elektrodenanordnung
- 66: Elektrodenanordnung
- 67: Aufnahmebereich
- 68: Elektroden
- 69: Elektroden
- 70: Kontaktpunkte
- 71: Kontaktpunkte
- 72: Kontaktpunkte
- 73: Kontaktpunkte
- 74: Elektrodenanordnung
- 75: Elektrodenanordnung
- 76: Aufnahmebereich
- 77: Elektrodenanordnung
- 78: Elektrodenanordnung
- 79: Sektion
- 80: Sektion
- 81: Kontaktpunkte
- 82: Kontaktpunkte

## Patentansprüche

1. Verfahren zur Beaufschlagung von adhärenten Zellen, die auf dem Boden eines Reaktionsraums haften, mit mindestens einem elektrischen Feld, bei dem das elektrische Feld durch das Anlegen einer Spannung an mindestens zwei aktive Elektroden (48, 56, 63) erzeugt wird, wobei mindestens drei Elektroden (48, 49, 56, 57, 63, 64) vorgesehen sind, von denen beim Anlegen der Spannung zur Erzeugung eines ersten elektrischen Feldes mindestens zwei Elektroden aktive Elektroden (48, 63) sind, und wobei mindestens ein zweites elektrisches Feld erzeugt wird, **dadurch gekennzeichnet, dass** beim Anlegen der Spannung zur Erzeugung des zweiten elektrischen Feldes mindestens eine der zwei zuvor aktiven Elektroden (48, 63) eine potentialfreie Elektrode (57, 64) ist, wobei so viele elektrische Felder erzeugt werden, dass alle beim Erzeugen des ersten elektrischen Feldes aktiven Elektroden (48, 63) während des Erzeugens der weiteren elektrischen Felder jeweils mindestens einmal potentialfreie Elektroden (57, 64) sind.

2. Verfahren nach Anspruch 1, **dadurch gekennzeichnet, dass** jedes elektrische Feld jeweils durch mindestens einen Spannungsimpuls, vorzugsweise mindestens zwei Spannungsimpulse, erzeugt wird und/oder dass die mindestens zwei elektrischen Felder jeweils durch mindestens einen Spannungsimpuls erzeugt werden, wobei der zeitliche Abstand zwischen den Spannungsimpulsen mindestens 500 ms, vorzugsweise mindestens 1 s, insbesondere 1 bis 10 s, beträgt.

3. Verfahren nach Anspruch 1 oder 2, **dadurch gekennzeichnet, dass** mehrere Reaktionsräume vorgesehen sind, in denen jeweils adhärente Zellen mit den elektrischen Feldern beaufschlagt werden, wobei der zeitliche Abstand zwischen zwei Spannungsimpulsen zur Erzeugung zweier elektrischer Felder in einem Reaktionsraum dazu genutzt wird, mindestens ein elektrisches Feld in mindestens einem weiteren Reaktionsraum zu erzeugen.

4. Verfahren nach einem der Ansprüche 1 bis 3, **dadurch gekennzeichnet, dass** die Elektroden (48, 49, 56, 57, 63, 64) und die adhärenten Zellen voneinander beabstandet sind und dass der Abstand zwischen den Zellen und den Elektroden (48, 49, 56, 57, 63, 64) auf einen empirisch ermittelten Wert eingestellt wird.

5. Vorrichtung (58), insbesondere zur Durchführung des Verfahrens nach einem der Ansprüche 1 bis 4, die mindestens drei Elektroden (63, 64) umfasst, welche über mindestens eine Schaltvorrichtung (59, 60, 61, 62) mit mindestens einer Spannungsquelle verbunden sind, **dadurch gekennzeichnet, dass** mindestens fünf Elektroden (63, 64) über vier Schaltvorrichtungen (59, 60, 61, 62) mit der mindestens einen Spannungsquelle verbunden sind, wobei mindestens zwei Elektroden (63, 64) über eine gemeinsame Schaltvorrichtung (59, 60, 61, 62) mit der Spannungsquelle verbunden sind, und wobei zwei Gruppen von Elektroden (63, 64) mit jeweils mindestens zwei Elektroden (63, 64) vorgesehen sind, die jeweils über zwei gemeinsame Schaltvorrichtungen (59, 60, 61, 62) mit der Spannungsquelle verbunden sind.

6. Vorrichtung nach Anspruch 5, **dadurch gekennzeichnet, dass** insgesamt mindestens sieben, vorzugsweise 9-21, Elektroden (63, 64) vorgesehen sind.

7. Vorrichtung nach Anspruch 5 oder 6, **dadurch gekennzeichnet, dass** die Elektroden (63, 64) zum Einsetzen in mindestens einen Reaktionsraum vorgesehen sind.

8. Vorrichtung nach Anspruch 7, **dadurch gekennzeichnet, dass** der Reaktionsraum Teil einer Multiwell-Platte ist.

9. Verwendung der Vorrichtung nach einem der Ansprüche 5 bis 8 zum Beaufschlagen von adhärenten Zellen mit mindestens einem elektrischen Feld, insbesondere zur Elektroporation oder Elektrofusion von adhärenten Zellen.

## Claims

1. Method for applying at least one electric field to adherent cells adhering to the bottom of a reaction space, in which the electric field is generated by applying a voltage to at least two active electrodes (48, 56, 63), wherein at least three electrodes (48, 49, 56, 57, 63, 64) are provided, at least two of these electrodes are active electrodes (48, 63) when the voltage is applied for generating a first electric field, and wherein at least a second electric field is generated, **characterized in that** at least one of the two former active electrodes (48, 63) is a floating electrode (57, 64) when the voltage is applied for generating the second electric field, wherein as many electric fields as necessary are generated during generation of further electric fields to make at least once floating electrodes (57, 64) out of all electrodes (48, 63) that have been active when the first electric field was generated.

2. Method according to claim 1, **characterized in that** each electrical field is respectively generated by at least one voltage pulse, preferably at least two voltage pulses, and/or that the at least two electrical fields are respectively generated by at least one voltage pulse wherein the time gap between the voltage pulses is at least 500 ms, preferably at least 1 s, in particular 1 to 10 s.

3. Method according to any one of claims 1 or 2, **characterized in that** several reaction spaces are provided within which the electric fields are respectively applied to adherent cells, wherein the time gap between two voltage pulses for generating two electric fields within one reaction space is utilized to generate at least one electric field within at least one further reaction space.

4. Method according to any one of claims 1 to 3, **characterized in that** the electrodes (48, 49, 56, 57, 63, 64) and the adherent cells are separated from each other and that the distance between the cells and the electrodes (48, 49, 56, 57, 63, 64) is adjusted to an empirically determined value.

5. Device (58), in particular for accomplishing the method according to any one of claims 1 to 4, comprising at least three electrodes (63, 64) which are connected to at least one voltage source via at least one switching device (59, 60, 61, 62), **characterized in that** at least five electrodes (63, 64) are connected to the at least one voltage source via four switching devices (59, 60, 61, 62), wherein at least two electrodes (63, 64) are connected to the voltage source via a shared switching device (59, 60, 61, 62), and wherein two groups of electrodes (63, 64) comprising at least two electrodes (63, 64) are provided, each of which being connected to the voltage source via two shared switching devices (59, 60, 61, 62).

6. Device according to claim 5, **characterized in that** a total of at least seven, preferably 9-21, electrodes (63, 64) are provided.

7. Device according to claim 5 or 6, **characterized in that** the electrodes (63, 64) are provided for insertion into at least one reaction space.

8. Device according to claim 7, **characterized in that** the reaction space is part of a multiwall plate.

9. Use of the device according to any one of claims 5 to 8 for applying at least one electric field to adherent cells, in particular for electroporation and electrofusion of adherent cells.

## Revendications

1. Procédé de sollicitation de cellules adhérentes, qui adhèrent au fond d'un espace de réaction, avec au moins un champ électrique, pour lequel le champ électrique est produit par l'application d'une tension à au moins deux électrodes actives (48, 56, 63), sachant qu'au moins trois électrodes (48, 49, 56, 57, 63, 64) sont prévues, parmi lesquelles lors de l'application de la tension pour produire un premier champ électrique au moins deux électrodes sont des électrodes actives (48, 63) et sachant qu'au moins un deuxième champ électrique est produit, **caractérisé en ce que** lors de l'application de la tension pour produire le deuxième champ électrique au moins une des deux électrodes préalablement actives (48, 63) est une électrode sans potentiel (57, 64), sachant que beaucoup de champs électriques sont produits de telle manière que toutes les électrodes actives (48, 63) lors de la production du premier champ électrique, sont au moins une fois des électrodes sans potentiel (57, 64) pendant la production des autres champs électriques.

2. Procédé selon la revendication 1, **caractérisé en ce que** chaque champ électrique est produit respectivement par au moins une impulsion de tension, de préférence au moins deux impulsions de tension et/ou **en ce qu'**au moins les deux champs électriques sont produits respectivement avec au moins une impulsion de tension, sachant que l'intervalle de temps entre les impulsions de tension est au moins de 500 ms, de préférence au moins de 1 s, en particulier de 1 à 10 s.

3. Procédé selon la revendication 1 ou 2, **caractérisé en ce que** plusieurs espaces de réaction sont prévus dans lesquels respectivement des cellules adhérentes sont sollicitées avec le champ électrique, sachant que l'intervalle de temps entre deux impulsions de tension pour produire deux champs électriques dans un espace de réaction est utilisé à cet effet pour produire au moins un champ électrique dans au moins un autre espace de réaction.

4. Procédé selon l'une quelconque des revendications 1 à 3, **caractérisé en ce que** les électrodes (48, 49, 56, 57, 63, 64) et les cellules adhérentes sont à distance les unes des autres et **en ce que** l'intervalle entre les cellules et les électrodes (48, 49, 56, 57, 63, 64) est réglé sur une valeur déterminée de façon empirique.

5. Procédé (58), en particulier pour exécuter le procédé selon l'une quelconque des revendications 1 à 4, qui comprend au moins trois électrodes (63, 64), lesquelles sont reliées par au moins un dispositif de connexion (59, 60, 61, 62) à au moins une source tension, **caractérisé en ce qu'**au moins cinq électrodes (63, 64) sont reliées par quatre dispositifs de connexion (59, 60, 61, 62) à au moins une source tension, sachant qu'au moins deux électrodes (63, 64) sont reliées par un dispositif de connexion commun (59, 60, 61, 62) à la source de tension et sachant que deux groupes d'électrodes (63, 64) sont prévus avec respectivement au moins deux électrodes (63, 64), qui sont reliées respectivement par deux dispositifs de connexion communs (59, 60, 61, 62) à la source de tension.

6. Dispositif selon la revendication 5, **caractérisé en ce qu'**au total au moins sept, de préférence 9-21, électrodes (63, 64) sont prévues.

7. Dispositif selon la revendication 5 ou 6, **caractérisé en ce que** les électrodes (63, 64) sont prévues pour utilisation dans au moins un espace de réaction.

8. Dispositif selon la revendication 7, **caractérisé en ce que** l'espace de réaction fait partie d'une plaque de Multiwell.

9. Utilisation du dispositif selon l'une quelconque des revendications 5 à 8 pour la sollicitation de cellules adhérentes avec au moins un champ électrique, en particulier pour l'extrapolation ou l'électroporation de cellules adhérentes.
